# EUROPEAN PATENT SPECIFICATION

(11) **EP 1 727 620 B1**
(45) Date of publication and mention of the grant of the patent: **29.08.2007**
(21) Application number: 05708733.0
(22) Date of filing: 09.03.2005
(51) Int. Cl.: B01J 31/24

(54) **C1-SYMMETRIC BISPHOSPHINE LIGANDS AND THEIR USE IN THE ASYMMETRIC SYNTHESIS OF PREGABALIN**
C1-SYMMETRISCHE BISPHOSPHINLIGANDEN UND DEREN VERWENDUNG BEI DER ASYMMETRISCHEN SYNTHESE VON PREGABALIN
LIGANDS DE BISPHOSPHINE C1-SYMMETRIQUES ET LEUR UTILISATION DANS LA SYNTHESE ASYMETRIQUE DE PREGABALINE

(30) Priority: 12.03.2004 US 552586 P; 09.07.2004 US 586512 P
(43) Date of publication of application: 06.12.2006
(73) Proprietor: Warner-Lambert Company LLC, New York, NY 10017 (US)
(72) Inventor: BAO, Jian, Chesterfield, MO 63017 (US); BEYLIN, Vladimir Genukh, 2800 Plymouth Road, Ann Arbor, MI 48105 (US); GREENE, Derek Joseph, 2800 Plymouth Road, Ann Arbor, MI 48105 (US); HOGE, Garrett, 2800 Plymouth Road, Ann Arbor, MI 48105 (US); KISSEL, William Scott, 2800 Plymouth Road, Ann Arbor, MI 48105 (US); MARLATT, Mark Eugene, 2800 Plymouth Road, Ann Arbor, MI 48105 (US); PFLUM, Derek Andrew, 2800 Plymouth Road, Ann Arbor, MI 48105 (US); WU, He-Ping, Edison, NJ 08820 (US)
(74) Representative: Rudge, Andrew John
(86) International application number: PCT/IB2005/000642
(87) International publication number: WO 2005/087370

(56) References cited:
- US-A1- 2002 143 214
- HOGE, GARRETT: "Synthesis of Both Enantiomers of a P-Chirogenic 1,2-Bisphospholanoethane Ligand via Convergent Routes and Application to Rhodium-Catalyzed Asymmetric Hydrogenation of CI-1008 (Pregabalin)" JOURNAL OF THE AMERICAN CHEMICAL SOCIETY , 125(34), 10219-10227 CODEN: JACSAT; ISSN: 0002-7863, 2003, XP002326305
- HOGE, GARRETT ET AL: "Highly Selective Asymmetric Hydrogenation Using a Three Hindered Quadrant Bisphosphine Rhodium Catalyst" JOURNAL OF THE AMERICAN CHEMICAL SOCIETY , 126(19), 5966-5967 CODEN: JACSAT; ISSN: 0002-7863, 2004, XP002326306

## Description

### FIELD OF INVENTION

This invention relates to C₁-symmetric bisphosphine ligands and corresponding catalysts, and to their use in asymmetric syntheses, including the enantioselective hydrogenation of prochiral olefins to prepare pharmaceutically useful compounds, including (*S*)-(+)-3-(aminomethyl)-5-methyl-hexanoic acid, 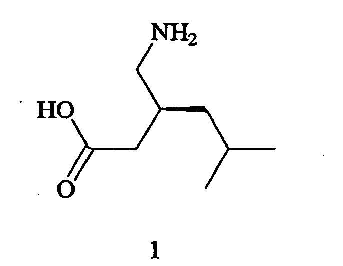 which is commonly known as pregabalin.

### DISCUSSION

Chiral phosphine ligands have played a significant role in the development of novel transition metal catalyzed asymmetric reactions to produce enantiomeric excess of compounds with desired activities. The first successful attempts at asymmetric hydrogenation of eneamide substrates were accomplished in the late 1970s using chiral bisphosphines as transition metal ligands. See, e.g., B. D. Vineyard et al., J. Am. Chem. Soc. 99(18):5946-52 (1977); W. S. Knowles et al., J. Am. Chem. Soc. 97(9):2567-68 (1975). Since these first published reports, there has been an explosion of research related to the synthesis of new chiral bisphosphine ligands for asymmetric hydrogenations and other chiral catalytic transformations. See I. Ojima, ed., Catalytic Asymmetric Synthesis (1993); D. J. Ager, ed., Handbook of Chiral Chemicals (1999).

Some of the most efficient and broadly useful ligands developed for asymmetric hydrogenation include BPE ligands (e.g., (*R,R*)-Et-BPE or (+)-1,2-bis((2*R*,5*R*)-2,5-diethylphospholano)ethane); DuPhos ligands (e.g., (*R,R*)-Me-DUPHOS or (-)-1,2-bis((2R,5R)-2,5-dimethylphospholano)benzene); and BisP* ligand ((S,S)-1,2-bis(t-butylmethylphosphino)ethane). See, e.g., M. J. Burk, Chemtracts 11(11):787-802 (1998); M. J. Burk et al., Angew Chem., Int. Ed. 37(13/14):1931-33 (1998); M. J. Burk, et al., J. Org. Chem. 63(18):6084-85 (1998); M. J. Burket al., J. Am. Chem. Soc. 120(18):4345-53 (1998); M. J. Burk et al., J. Am. Chem. Soc. 117(15):4423-24 (1995); M. J. Burk et al., J. Am. Chem. Soc. 115(22):10125-38 (1993); W. A. Nugent et al., Science 259(5094):479-83 (1993); M. J. Burk et al., Tetrahedron: Asymmetry 2(7):569-92 (1991); M. J. Burk, J. Am. Chem. Soc. 113(22):8518-19 (1991); T. Imamoto et al., J. Am. Chem. Soc. 120(7):1635-36 (1998); G. Zhu et al., J. Am. Chem. Soc. 119(7):1799-800 (1997).

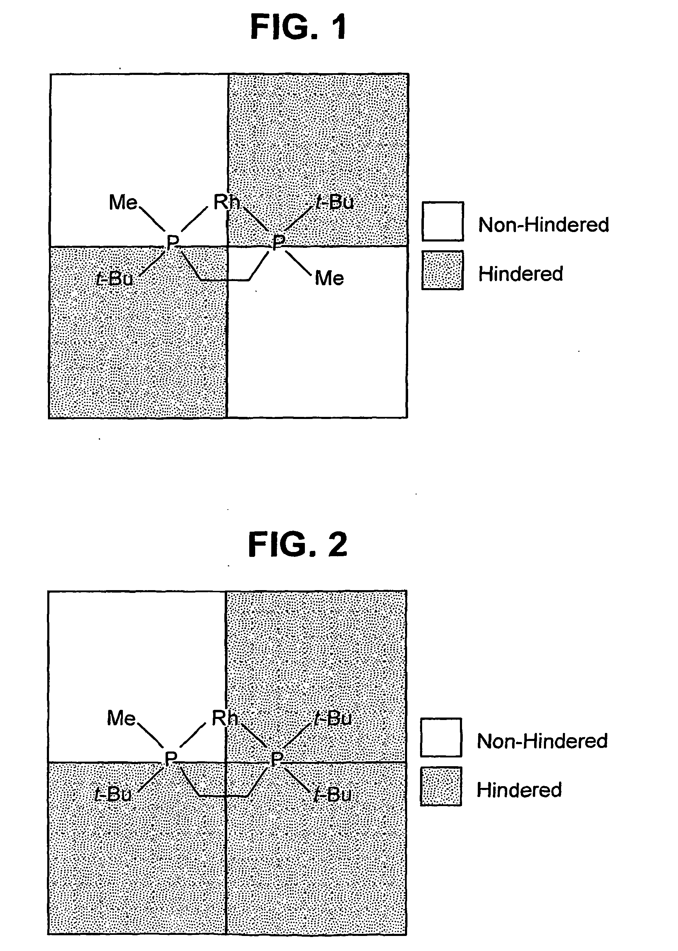

The success of BPE, DUPHOS, BisP* and related ligands in asymmetric hydrogenation reactions has been attributed, among other factors, to rigidity in their C₂-symmetric structure. As shown in FIG. 1, dividing the spatial area of a phosphine ligand structure, such as BisP*, into four quadrants results in alternating hindered and unhindered quadrants when bound to a transition metal (e.g., Rh). This structural motif has driven the design of bisphosphine ligands and corresponding catalysts for asymmetric hydrogenation of certain substrates-including eneamides, enol esters, and succinates-and may have delayed the development of non-C₂-symmetric (i.e., C₁-symmetric) bisphosphine ligands.

Researchers have recently described C₁-symmetric bisphosphine ligands and corresponding catalysts, which are useful in asymmetric transformations, including enantioselective hydrogenation reactions. See, e.g., commonly assigned U.S. Patent Application No. 2002/0143214 A1, published October 3, 2002, and commonly assigned U.S. Patent Application No. 2003/0073868, published April 17, 2003. As shown in FIG. 2, these ligands, as represented by (*t*-butyl-methyl-phosphanyl)-(dit-butyl-phosphanyl)-ethane display a three-hindered quadrant steric environment when bound to a transition metal, such as Rh. However, cohesive models of C₁-symmetric bisphosphine ligands and corresponding catalysts, which relate their steric environments to enantioselectivity during hydrogenation remain elusive. See, for example, H. Blaser et al., Topics in Catalysis 19:3 (2002); A. Ohashi et al., European Journal of Organic Chemistry 15:2535 (2002); K. Matsumura et al., Advanced Synthesis & Catalysis 345:180 (2003).

Pregabalin, (S)-(+)-3-aminomethyl-5-methyl-hexanoic acid, binds to the alpha-2-delta (α2δ) subunit of a calcium channel, and is related to the endogenous inhibitory neurotransmitter γ-aminobutyric acid (GABA), which is involved in the regulation of brain neuronal activity. Pregabalin exhibits anti-seizure activity, as described in U.S. Patent No. 5,563,175 to R. B. Silverman et al., and is thought to be useful for treating, among other conditions, pain, physiological conditions associated with psychomotor stimulants, inflammation, gastrointestinal damage, alcoholism, insomnia, and various psychiatric disorders, including mania and bipolar disorder. See, respectively, U.S. Patent No. 6,242,488 to L. Bueno et al., U.S. Patent No. 6,326,374 to L. Magnus & C. A. Segal, and U.S. Patent No. 6,001,876 to L. Singh; U.S. Patent No. 6,194,459 to H. C. Akunne et al.; U.S. Patent No. 6,329,429 to D. Schrier et al.; U.S. Patent No. 6,127,418 to L. Bueno et al.; U.S. Patent No. 6,426,368 to L Bueno et al.; U.S. Patent No. 6,306,910 to L. Magnus & C. A. Segal; and U.S. Patent No. 6,359,005 to A. C. Pande.

Pregabalin has been prepared in various ways. Typically, a racemic mixture of 3-(aminomethyl)-5-methyl-hexanoic acid is synthesized and subsequently resolved into its R- and S-enantiomers. Such methods may employ an azide intermediate (e.g., U.S. Patent No. 5,563,175 to R. B. Silverman et al.), a malonate intermediate (e.g., U.S. Patent No. 6,046,353 to T. M. Grote et al., U.S. Patent No. 5,840,956 to T. M. Grote et al., and U.S. Patent No. 5,637,767 to T. M. Grote et al.), or Hofman synthesis (U.S. Patent No. 5,629,447 to B. K. Huckabee & D. M. Sobieray, and U.S. Patent No. 5,616,793 to B. K. Huckabee & D. M. Sobieray). In each of these methods, the racemate is reacted with a chiral acid (a resolving agent) to form a pair of diastereoisomeric salts, which are separated by known techniques, such as fractional crystallization and chromatography. These methods thus involve significant processing beyond the preparation of the racemate, which along with the resolving agent, adds to production costs. Moreover, the undesired R-enantiomer is frequently discarded since it cannot be efficiently recycled, thereby reducing the effective throughput of the process by 50%.

In addition, pregabalin has been synthesized directly using a chiral auxiliary, (4*R*,5*S*)-4-methyl-5-phenyl-2-oxazolidinone. See, e.g., U.S. Patent Nos. 6,359,169, 6,028,214, 5,847,151, 5,710,304, 5,684,189, 5,608,090, and 5,599,973, all to Silverman et al. Although these methods provide pregabalin in high enantiomeric purity, they are less desirable for large-scale synthesis because they employ costly reagents (e.g., the chiral auxiliary) that are difficult to handle, as well as special cryogenic equipment to reach required operating temperatures, which can be as low as -78°C.

U.S. Patent Application 2003/0212290 A1 describes a method of making pregabalin via asymmetric hydrogenation of a cyano-substituted olefin to produce a chiral cyano precursor of (*S*)-3-(aminomethyl)-5-methylhexanoic acid. The cyano precursor is subsequently reduced to yield pregabalin. The application discloses the use of various C₂-synunetric bisphosphine ligands, including (*R*,*R*)-Me-DUPHOS, which result in substantial enrichment of pregabalin over (*R*)-3-(aminomethyl)-5-methylhexanoic acid.

Although the method disclosed in U.S. Patent Application 2003/0212290 A1 represents a commercially viable method for preparing pregabalin, further improvements would be desirable for many reasons. C₂-symmetric bisphosphine ligands, including the proprietary ligand (R,R)-Me-DUPHOS, are often difficult to prepare because they possess two chiral centers, which adds to their cost. Furthermore, although the chiral catalysts disclosed in U.S. Patent Application 2003/0212290 A1 generate the cyano precursor of pregabalin in good enantiomeric excess (in some cases, equal to about 95% ee or greater), higher enantioselectivity (equal to about 98% ee or greater) would be beneficial. Additionally, chiral catalysts capable of being used at higher substrate-to-catalyst ratios (s/c) would be beneficial since they would permit, for a given catalyst loading or substrate concentration, higher substrate concentrations or lower catalyst loadings. Higher substrate concentrations would result in increased process throughput and therefore lower unit production costs. Similarly, lower catalyst loadings would result in substantially lower unit production costs.

### SUMMARY OF THE INVENTION

The present invention provides materials and methods for preparing pregabalin (Formula 1) and structurally related compounds. The claimed methods employ novel chiral catalysts, each of which comprises a C₁-symmetric bisphosphine ligand bound to a transition metal (e.g., rhodium) through phosphorus atoms. The claimed invention provides many advantageous over existing methods for preparing pregabalin and similar compounds. For example, the C₁-symmetric bisphosphine ligands have a single stereogenic center, which should make the ligands and their corresponding chiral catalysts relatively inexpensive to prepare. Moreover, and as indicated in the examples below, the claimed invention can generate a chiral cyano precursor of pregabalin with higher enantioselectivity (about 98% ee or greater) than known methods. As also shown in the examples below, the novel chiral catalysts may be used at higher substrate-to-catalyst ratios (s/c) than known catalysts, which should lead to substantially lower unit production costs.

One aspect of the present invention provides a method of making a desired enantiomer of a compound of Formula 2, 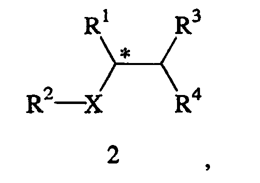 or a pharmaceutically acceptable complex, salt, solvate or hydrate thereof. In Formula 2,
- R¹: is C₁₋₆ alkyl, C₁₋₇ alkanoylamino, C₁₋₆ alkoxycarbonyl, C₁₋₆ alkoxycarbonylamino, amino, amino-C₁₋₆ alkyl, C₁₋₆ alkylamino, cyano, cyano-C₁₋₆ alkyl, carboxy, or -CO₂-Y;
- R²: is C₁₋₇ alkanoyl, C₁₋₆ alkoxycarbonyl, carboxy, or -CO₂-Y;
- R³ and R⁴: are independently hydrogen atom, C₁₋₆ alkyl, C₃₋₇ cycloalkyl, aryl, or aryl-C₁₋₆ alkyl, or R³ and R⁴ together are C₂₋₆ alkanediyl;
- X: is -NH-, -O-, -CH₂-, or a bond;
- Y: is a cation, and the asterisk designates a stereogenic (chiral) center.
The method includes the steps of (a) reacting a prochiral substrate (olefin) of Formula 3, 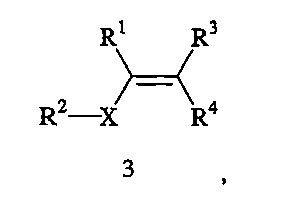 with hydrogen in the presence of a chiral catalyst to yield the compound of Formula 2; and (b) optionally converting the compound of Formula 2 into a pharmaceutically acceptable complex, salt, solvate or hydrate. Substituents R¹, R², R³, R⁴, and X in Formula 3 are as defined in Formula 2. The chiral catalyst comprises a chiral ligand bound to a transition metal through phosphorus atoms, and has a structure represented by Formula 4,

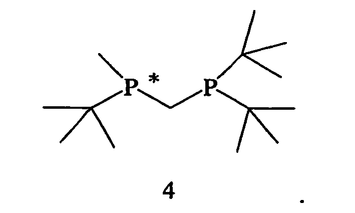

Generally, the method may be used to produce the desired enantiomer of the compound of Formula 2 with an ee of about 95 % or greater, and in some cases, with an ee of about 99 % or greater. Useful prochiral substrates include 3-cyano-5-methyl-hex-3-ennoic acid or base addition salts thereof, such as 3-cyano-5-methyl-hex-3-enoate t-butyl-ammonium salt. Other useful prochiral substrates include those in which Y is a Group 1 (alkali) metal ion, a Group 2 (alkaline earth) metal ion, a primary ammonium ion, or a secondary ammonium ion.

A particularly useful chiral catalyst includes the chiral ligand of Formula 4, which is bound to rhodium through the phosphorus atoms. Another particularly useful chiral catalyst includes an enantiomer of the bisphosphine ligand of Formula 4, which has a structure represented by Formula 5, 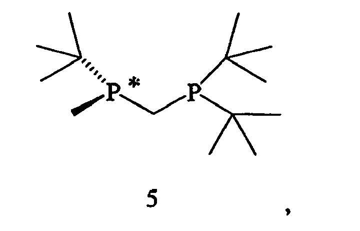 and an ee of about 95 % or greater. An especially useful chiral catalyst includes an enantiomer of the bisphosphine ligand of Formula 4 having a structure represented by Formula 5 and ee of about 99 % or greater.

Another aspect of the present invention provides a method of making pregabalin or (*S*)-(+)-3-(aminomethyl)-5-methyl-hexanoic acid (Formula 1) or a pharmaceutically acceptable complex, salt, solvate or hydrate thereof. The method includes the steps of (a) reacting a compound of Formula 6, 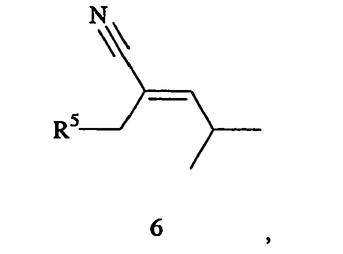 its corresponding Z-isomer, or a mixture thereof, with H₂ (hydrogen) in the presence of a chiral catalyst to yield a compound of Formula 7, 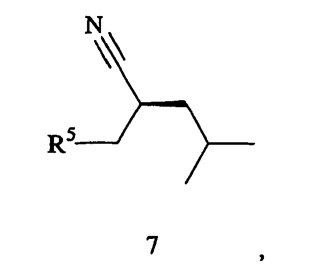 wherein R⁵ is a carboxy group or -CO₂-Y, Y is a cation, and the chiral catalyst comprises a chiral ligand (Formula 4) bound to a transition metal through phosphorus atoms; (b) reducing a cyano moiety of the compound of Formula 7 to yield a compound of Formula 8, 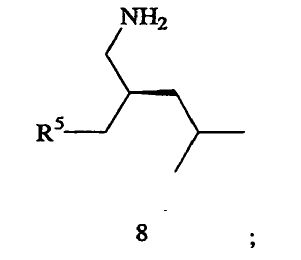 (c) optionally treating the compound of Formula 8 with an acid to yield pregabalin; and (d) optionally converting the compound of Formula 8 or Formula 1 to a pharmaceutically acceptable complex, salt, solvate or hydrate.

The method may be used to produce pregabalin having an ee of about 95 % or greater, or having an ee of about 99 % or greater, and in some cases having an ee of about 99.9 % or greater. Useful prochiral substrates (Formula 6) include a base addition salt of 3-cyano-5-methyl-hex-3-enoic acid, such as 3-cyano-5-methyl-hex-3-enoate t-butyl-ammonium salt. Other useful prochiral substrates include those in which Y in Formula 6 is a Group 1 metal ion, a Group 2 metal ion, a primary ammonium ion, or a secondary ammonium ion. A particularly useful chiral catalyst includes the chiral ligand of Formula 4, which is bound to rhodium through the phosphorus atoms. Another particularly useful chiral catalyst includes an enantiomer of the bisphosphine ligand of Formula 4, which has a structure represented by Formula 5 (above), and an ee of about 95 % or greater. An especially useful chiral catalyst includes an enantiomer of the bisphosphine ligand of Formula 4 having a structure represented by Formula 5 and ee of about 99 % or greater.

Still another aspect of the present invention provides a method of making a desired enantiomer of the compound of Formula 4. The method includes the steps of (a) reacting a compound of Formula 9, 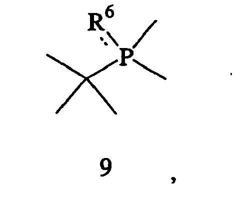 with a compound of Formula 10, 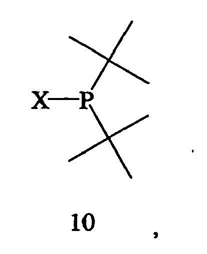 to yield a compound of Formula 11, 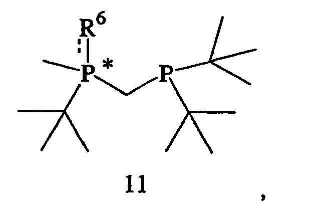 in which the compound of Formula 9 is treated with a base prior to reaction with the compound of Formula 10, X is a leaving group, and R⁶ is BH₃, sulfur or oxygen; (b) reacting the compound of Formula 11 with a borane, with sulfur, or with oxygen to yield a compound of Formula 12, 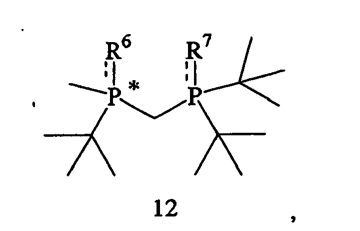 wherein R⁷ is the same as or different than R⁶ and is BH₃, sulfur, or oxygen; and (c) removing R⁶ and R⁷ from the compound of Formula 12 to yield the compound of Formula 4.

The claimed method is particularly useful for making the R-enantiomer of the compound of Formula 5, having an ee of about 80 %, about 90 %, about 95 % or about 99 % or greater. Typically, the compound of Formula 12 is resolved into separate enantiomers before removal of R⁶ and R⁷. Substituents R⁶ and R⁷ may be removed many different ways depending on the nature of the particular substituents. For instance, when R⁶ and R⁷ are each BH₃, they may be removed by reacting a compound of Formula 13, 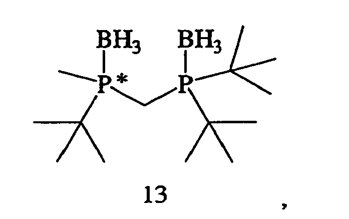 with an amine or an acid to yield the compound of Formula 4. Thus, for example, the compound of Formula 13 may be reacted with HBF₄•Me₂0, followed by base hydrolysis to yield the compound of Formula 4. Similarly, the compound of Formula 13 may be treated with DABCO, TMEDA, DBU, or Et₂NH, or combinations thereof to remove R⁶ and R⁷.

When both substituents are sulfur atoms, R⁶ and R⁷ may be removed using various techniques. One method includes the steps of (a) reacting a compound of Formula 14, 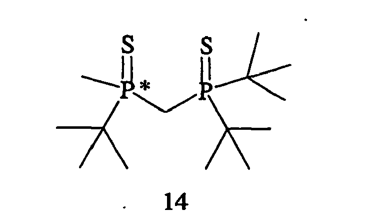 with R⁸OTf to yield a compound of Formula 15, 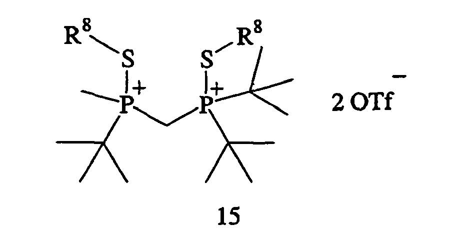 in which R⁸ is a C₁₋₄ alkyl; (b) reacting the compound of Formula 15 with a borohydride to yield the compound of Formula 13; and (c) reacting the compound of Formula 13 with an amine or an acid to yield the compound of Formula 4. A particularly useful R⁸ substituent is methyl and a particularly useful borohydride is LiBH₄.

Another method includes steps (a) and (b) above, and further includes the steps of (c) reacting the compound of Formula 13 with HCl to yield a compound of Formula 15, 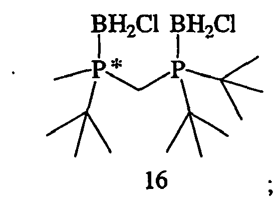 and
(d) reacting the compound of Formula 16 with an amine or an acid to yield the compound of Formula 4. When both substituents are sulfur or oxygen, R⁶ and R⁷ may also be removed by treating the compound of Formula 12 with a reducing agent, including a perchloropolysilane such as hexachlorodisilane.

Yet another aspect of the present invention provides a method of making a catalyst or pre-catalyst comprised of a chiral ligand bound to a transition metal through phosphorus atoms, the chiral ligand having a structure represented by Formula 4. The method includes the steps of (a) removing both R⁹ substituents from a compound of Formula 17, 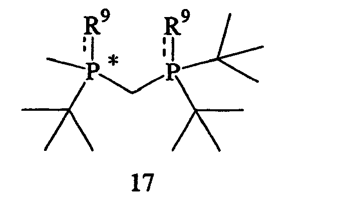 to yield a compound of Formula 4, wherein R⁹ is BH₃, sulfur, or oxygen; and (b) binding the compound of Formula 4 to a transition metal (e.g., rhodium). Step (b) may include reacting the compound of Formula 4 with a complex of Formula 18,

[Rh(L¹)ₘ(L²)ₙ]A_{P}, 18

in which
- L¹: is a diene selected from COD, norbornadiene, or 2,5-dimethyl-hexa-1,5-diene;
- L²: is an anionic ligand selected from Cl⁻, Br⁻, I⁻, ⁻CN, ⁻OR¹⁰, or ⁻R¹⁰, or a neutral σ-donor ligand selected from NR¹⁰R¹¹R¹², R¹⁰OR¹¹, R¹⁰SR¹¹, CO, or NCR¹⁰, wherein R¹⁰, R¹¹, and R¹² are independently H or C₁₋₆ alkyl;
- A: is an anion selected from OTf⁻, PF₆⁻, BF₄⁻, SbF₆⁻, or ClO₄⁻ ;
- m: is an integer from 0 to 2, inclusive;
- n: is an integer from 0 to 4, inclusive; and
- p: is a positive odd integer such that 4xm + 2xn + p = 9.

A further aspect of the present invention provides compounds of Formula 19, 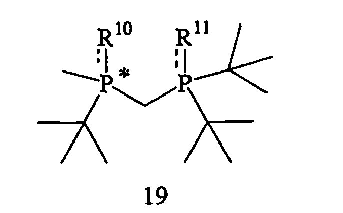 in which R¹⁰ and R¹¹ are independently BH₃, BH₂Cl, sulfur, oxygen, C₁₋₄ alkylthio, or absent, and subject to the proviso that R¹⁰ and R¹¹ are not both BH₃.

Useful compounds of Formula 19 include those in which R¹⁰ and R¹¹ are absent and those having R-absolute stereochemical configuration with an ee of about 95 % or with an ee of 99 % or greater. Other useful compounds of Formula 19 include those in which R¹⁰ and R¹¹ are the same, and are each oxygen, sulfur or C₁₋₄ alkylthio, and those having R-absolute stereochemical configuration with an ee of about 95 % or greater or with an ee of about 99 % or greater. Particularly useful compounds represented by Formula 19 thus include:
2-{[(di-*t*-butyl-phosphanyl)-methyl]-methyl-phosphanyl}-2-methyl-propane;
*(R)-* 2- {[(di-*t*-butyl-phosphanyl)-methyl]-methyl-phosphanyl}-2-methylpropane;
(*S*)-2-{[(di-*t*-butyl-phosphanyl)-methyl]-methyl-phosphanyl}-2-methylpropane;
2-((di-*t*-butyl-phosphinothioylmethyl)-methyl-phosphinothioyl]-2-methylpropane;
(R)-2-[(di-t-butyl-phosphinothioylmethyl)-methyl-phosphinothioyl]-2-methylpropane;
(*S*)-2-[(di-*t*-butyl-phosphinothioylmethyl)-methyl-phosphinothioyl]-2-methylpropane;
2-[(di-*t*-butyl-phosphinoylmethyl)-methyl-phosphinoyl]-2-methyl-propane;
(*R*)-2-[(di-*t*-butyl-phosphinoylmethyl)-methyl-phosphinoyl]-2-methylpropane;
(*S*)-2-[(di-*t*-butyl-phosphinoylmethyl)-methyl-phosphinoyl]-2-methylpropane;
(di-*t*-butyl-methylthio-phosphoniumyl-methyl)-*t*-butyl-methyl-methylthio-phosphonium;
(*R*)-(di-*t*-butyl-methylthio-phosphoniumyl-methyl)-*t*-butyl-methyl-methylthio-phosphonium; or
(*S*)-(di-*t*-butyl-methylthio-phosphoniumyl-methyl)-*t*-butyl-methyl-methylthio-phosphonium.

An additional aspect of the present invention provides a catalyst or pre-catalyst comprising a chiral ligand bound to a transition metal through phosphorus atoms. The chiral ligand has a structure represented by Formula 4.

A particularly useful chiral catalyst or pre-catalyst includes rhodium bound to a bisphosphine ligand having a structure represented by Formula 5. Other useful chiral catalysts or pre-catalysts include the bisphosphine ligand having a structure represented by Formula 5 and an ee of about 95 % or greater. An especially useful chiral catalyst includes the bisphosphine ligand having a structure represented by Formula 5 and ee of about 99 % or greater. The catalyst or pre-catalyst may further include one or more dienes (e.g., COD) or halogen anions (e.g., Cl⁻) bound to the transition metal, and may include a counterion, such as OTf⁻, PF₆⁻, BF₄⁻, SbF₆⁻, or ClO₄⁻, or mixtures thereof.

A further aspect of the present invention provides method of making a desired enantiomer of a compound of Formula 32, 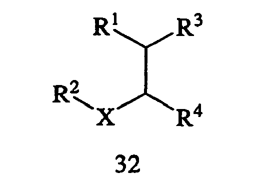 or a pharmaceutically acceptable complex, salt, solvate or hydrate thereof. The method comprises the steps of (a) reacting a compound of Formula 33, 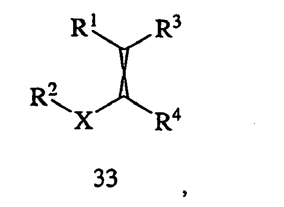 with hydrogen in the presence of a chiral catalyst to yield the compound of Formula 32; and (b) optionally converting the compound of Formula 32 into a pharmaceutically acceptable complex, salt, solvate or hydrate. Substituents R¹, R², R³, R⁴, and X in Formula 32 and Formula 33 are as defined in Formula 2; the chiral catalyst comprises a chiral ligand bound to a transition metal through phosphorus atoms, the chiral ligand having a structure represented by Formula 4, above. Useful compounds of Formula 32 include optically active β-amino acids that, like pregabalin, bind to the α2δ subunit of a calcium channel. These compounds, including their pharmaceutically acceptable complexes, salts, solvates and hydrates, are useful for treating pain, fibromyalgia, and a variety of psychiatric and sleep disorders. See, e.g., U.S. Patent Application No. 2003/0195251 A1 to Barta et al., the complete disclosure of which is herein incorporated by reference.

The scope of the present invention includes all pharmaceutically acceptable complexes, salts, solvates, hydrates, polymorphs, esters, amides, and prodrugs of the claimed and disclosed compounds, including compounds of Formula 1, 2, 8, and 32.

### BRIEF DESCRIPTION OF THE DRAWINGS

FIG. 1 depicts the spatial arrangement of a C₂-symmetric bisphosphine ligand (e.g., BisP*) when bound to a transition metal such as Rh.
FIG. 2 depicts the spatial arrangement of a C₁-symmetric bisphosphine ligand (e.g., (*t*-butyl-methyl-phosphanyl)-(di-*t-*butyl-phosphanyl)-ethane) when bound to a transition metal such as Rh.

### DETAILED DESCRIPTION

### DEFINITIONS AND ABBREVIATIONS

Unless otherwise indicated, this disclosure uses definitions provided below. Some of the definitions and formulae may include a "-" (dash) to indicate a bond between atoms or a point of attachment to a named or unnamed atom or group of atoms. Other definitions and formulae may include an "=" (equal sign) or "≡" (identity sign) to indicate a double bond or a triple bond, respectively. Certain formulae may also include one or more "*" (asterisks) to indicate stereogenic (chiral) centers, although the absence of asterisks does not indicate that the compound lacks one or more stereocenters. Such formulae may refer to the racemate or to individual enantiomers or diastereomers, which may or may not be substantially pure. Some formulae may also include a crossed double bond or a double either bond, to indicate a Z-isomer, an E-isomer, or a mixture of Z and E isomers.

"Substituted" groups are those in which one or more hydrogen atoms have been replaced with one or more non-hydrogen atoms or groups, provided that valence requirements are met and that a chemically stable compound results from the substitution.

"Alkyl" refers to straight chain and branched saturated hydrocarbon groups, generally having a specified number of carbon atoms (i.e., C₁₋₆ alkyl refers to an alkyl group having 1, 2, 3, 4, 5, or 6 carbon atoms). Examples of alkyl groups include, without limitation, methyl, ethyl, n-propyl, i-propyl, n-butyl, s-butyl, i-butyl, t-butyl, pent-1-yl, pent-2-yl, pent-3-yl, 3-methylbut-1-yl, 3-methylbut-2-yl, 2-methylbut-2-yl, 2,2,2-trimethyleth-1-yl, n-hexyl, and the like.

"Alkenyl" refers to straight chain and branched hydrocarbon groups having one or more unsaturated carbon-carbon bonds, and generally having a specified number of carbon atoms. Examples of alkenyl groups include, without limitation, ethenyl, 1-propen-1-yl, 1-propen-2-yl, 2-propen-1-yl, 1-buten-1-yl, 1-buten-2-yl, 3-buten-1-yl, 3-buten-2-yl, 2-buten-1-yl, 2-buten-2-yl, 2-methyl-1-propen-1-yl, 2-methyl-2-propen-1-yl, 1,3-butadien-1-yl, 1,3-butadien-2-yl, and the like.

"Alkynyl" refers to straight chain or branched hydrocarbon groups having one or more triple carbon-carbon bonds, and generally having a specified number of carbon atoms. Examples of alkynyl groups include, without limitation, ethynyl, 1-propyn-1-yl, 2-propyn-1-yl, 1-butyn-1-yl, 3-butyn-1-yl, 3-butyn-2-yl, 2-butyn-1-yl, and the like.

"Alkanediyl" refers to divalent straight chain and branched saturated hydrocarbon groups, generally having a specified number of carbon atoms. Examples include, without limitation, methylene, 1,2-ethanediyl, 1,3-propanediyl, 1,4-butanediyl, 1,5-pentanediyl, 1,6-hexanediyl, and the like.

"Alkanoyl" and "alkanoylamino" refer, respectively, to alkyl-C(O)- and alkyl-C(O)-NH-, where alkyl is defined above, and generally includes a specified number of carbon atoms, including the carbonyl carbon. Examples of alkanoyl groups include, without limitation, formyl, acetyl, propionyl, butyryl, pentanoyl, hexanoyl, and the like.

"Cycloalkyl" refers to saturated monocyclic and bicyclic hydrocarbon rings, generally having a specified number of carbon atoms that comprise the ring (i.e., C₃₋₇ cycloalkyl refers to a cycloalkyl group having 3, 4, 5, 6 or 7 carbon atoms as ring members). The cycloalkyl may be attached to a parent group or to a substrate at any ring atom, unless such attachment would violate valence requirements. Likewise, the cycloalkyl groups may include one or more non-hydrogen substituents unless such substitution would violate valence requirements. Useful substituents include, without limitation, alkyl, alkoxy, alkoxycarbonyl, and alkanoyl, as defined above, and hydroxy, mercapto, nitro, halogen, and amino.

Examples of monocyclic cycloalkyl groups include, without limitation, cyclopropyl, cyclobutyl, cyclopentyl, cyclohexyl, and the like. Examples of bicyclic cycloalkyl groups include, without limitation, bicyclo[1.1.0]butyl, bicyclo[1.1.1]pentyl, bicyclo[2.1.0]pentyl, bicyclo[2.1.1]hexyl, bicyclo[3.1.0]hexyl, bicyclo[2.2.1]heptyl, bicyclo[3.2.0]heptyl, bicyclo[3.1.1]heptyl, bicyclo[4.1.0]heptyl, bicyclo[2.2.2]octyl, bicyclo[3.2.1]octyl, bicyclo[4.1.1]octyl, bicyclo[3.3.0]octyl, bicyclo[4.2.0]octyl, bicyclo[3.3.1]nonyl, bicyclo[4.2.1]nonyl, bicyclo[4.3.0]nonyl, bicyclo[3.3.2]decyl, bicyclo[4.2.2]decyl, bicyclo[4.3.1]decyl, bicyclo[4.4.0]decyl, bicyclo[3.3.3]undecyl, bicyclo[4.3.2]undecyl, bicyclo[4.3.3]dodecyl, and the like.

"Cycloalkanoyl" refers to cycloalkyl-C(O)-, where cycloalkyl is defined above, and generally includes a specified number of carbon atoms, excluding the carbonyl carbon. Examples of cycloalkanoyl groups include, without limitation, cyclopropanoyl, cyclobutanoyl, cyclopentanoyl, cyclohexanoyl, cycloheptanoyl, and the like.

"Alkoxy," "alkoxycarbonyl," and "alkoxycarbonylamino," refer, respectively, to alkyl-O-, alkyl-O-C(O)-, and alkyl-O-C(O)-NH-, where alkyl is defined above. Examples of alkoxy groups include, without limitation, methoxy, ethoxy, *n*-propoxy, *i*-propoxy, *n*-butoxy, *s*-butoxy, *t*-butoxy, *n*-pentoxy, *s*-pentoxy, and the like.

"Alkylamino," "alkylaminocarbonyl," "dialkylaminocarbonyl," "alkylsulfonyl" "sulfonylaminoalkyl," and "alkylsulfonylaminocarbonyl" refer, respectively, to alkyl-NH-, alkyl-NH-C(O)-, alkyl₂-N-C(O)-, alkyl-S(O₂)-, HS(O₂)-NH-alkyl-, and alkyl-S(O)-NH-C(O)-, where alkyl is defined above.

"Aminoalkyl" and "cyanoalkyl" refer, respectively, to NH₂-alkyl and N≡C-alkyl, where alkyl is defined above.

"Halo," "halogen" and "halogeno" may be used interchangeably, and refer to fluoro, chloro, bromo, and iodo.

"Haloalkyl" and "haloalkanoyl" refer, respectively, to alkyl or alkanoyl groups substituted with one or more halogen atoms, where alkyl and alkanoyl are defined above. Examples of haloalkyl and haloalkanoyl groups include, without limitation, trifluoromethyl, trichloromethyl, pentafluoroethyl, pentachloroethyl, trifluoroacetyl, trichloroacetyl, pentafluoropropionyl, pentachloropropionyl, and the like.

"Hydroxyalkyl" and "oxoalkyl" refer, respectively, to HO-alkyl and O=alkyl, where allyl is defined above. Examples of hydroxyalkyl and oxoalkyl groups, include, without limitation, hydroxymethyl, hydroxyethyl, 3-hydroxypropyl, oxomethyl, oxoethyl, 3-oxopropyl, and the like.

"Aryl" and "arylene" refer to monovalent and divalent aromatic groups, respectively. Examples of aryl groups include, without limitation, phenyl, naphthyl, biphenyl, pyrenyl, anthracenyl, fluorenyl, and the like, which may be unsubstituted or substituted with 1 to 4 substituents. Such substituents include, without limitation, alkyl, alkoxy, alkoxycarbonyl, alkanoyl, and cycloalkanoyl, as defined above, and hydroxy, mercapto, nitro, halogen, and amino.

"Arylalkyl" refers to aryl-alkyl, where aryl and alkyl are defined above. Examples include, without limitation, benzyl, fluorenylmethyl, and the like.

"Arylalkanoyl" refers to aryl-alkanoyl, where aryl and alkanoyl are defined above. Examples include, without limitation, benzoyl, phenylethanoyl, phenylpropanoyl, and the like.

"Arylalkoxycarbonyl" refers to aryl-alkoxycarbonyl, where aryl and alkoxycarbonyl are defined above. Examples include, without limitation, phenoxycarbonyl, benzyloxycarbonyl (CBz), and the like.

"Heterocycle" and "heterocyclyl" refer to saturated, partially unsaturated, or unsaturated monocyclic or bicyclic rings having from 5 to 7 or from 7 to 11 ring members, respectively. These groups have ring members made up of carbon atoms and from 1 to 4 heteroatoms that are independently nitrogen, oxygen or sulfur, and may include any bicyclic group in which any of the above-defined monocyclic heterocycles are fused to a benzene ring. The nitrogen and sulfur heteroatoms may optionally be oxidized. The heterocyclic ring may be attached to a parent group or to a substrate at any heteroatom or carbon atom unless such attachment would violate valence requirements. Likewise, any of the carbon or nitrogen ring members may include a non-hydrogen substituent unless such substitution would violate valence requirements. Useful substituents include, without limitation, alkyl, alkoxy, alkoxycarbonyl, alkanoyl, and cycloalkanoyl, as defined above, and hydroxy, mercapto, nitro, halogen, and amino.

Examples of heterocycles include, without limitation, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazolinyl, carbazolyl, 4a*H*-carbazolyl, carbolinyl, chromanyl, chromenyl, cinnolinyl, decahydroquinolinyl, 2*H*, 6*H*-1,5,2-dithiazinyl, dihydrofuro[2,3-b]tetrahydrofuran, furanyl, furazanyl, imidazolidinyl, imidazolinyl, imidazolyl, 1*H-*indazolyl, indolenyl, indolinyl, indolizinyl, indolyl, 3*H*-indolyl, isobenzofuranyl, isochromanyl, isoindazolyl, isoindolinyl, isoindolyl, isoquinolinyl, isothiazolyl, isoxazolyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolidinyl, pyrimidinyl, phenanthridinyl, phenanthrolinyl, phenazinyl, phenothiazinyl, phenoxathiinyl, phenoxazinyl, phthalazinyl, piperazinyl, piperidinyl, pteridinyl, purinyl, pyranyl, pyrazinyl, pyrazolidinyl, pyrazolinyl, pyrazolyl, pyridazinyl, pyridooxazole, pyridoimidazole, pyridothiazole, pyridinyl, pyridyl, pyrimidinyl, pyrrolidinyl, pyrrolinyl, 2H-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thienothiazolyl, thienooxazolyl, thienoimidazolyl, thiophenyl, triazinyl, 1,2,3-triazolyl, 1,2,4-triazolyl, 1,2,5-triazolyl, 1,3,4-triazolyl, and xanthenyl.

"Heteroaryl" and "heteroarylene" refer, respectively, to monovalent and divalent heterocycles or heterocyclyl groups, as defined above, which are aromatic. Heteroaryl and heteroarylene groups represent a subset of aryl and arylene groups, respectively.

"Leaving group" refers to any group that leaves a molecule during a fragmentation process, including substitution reactions, elimination reactions, and addition-elimination reactions. Leaving groups may be nucleofugal, in which the group leaves with a pair of electrons that formerly served as the bond between the leaving group and the molecule, or may be electrofugal, in which the group leaves without the pair of electrons. The ability of a nucleofugal leaving group to leave depends on its base strength, with the strongest bases being the poorest leaving groups. Common nucleofugal leaving groups include nitrogen (e.g., from diazonium salts), sulfonates (including tosylates, brosylates, nosylates, and mesylates), triflates, nonaflates, tresylates, halide ions, carboxylate anions, phenolate ions, and alkoxides. Some stronger bases, such as NH₂⁻ and OH⁻ can be made better leaving groups by treatment with an acid. Common electrofugal leaving groups include the proton, CO₂, and metals.

"Enantiomeric excess" or "ee" is a measure, for a given sample, of the excess of one enantiomer over a racemic sample of a chiral compound and is expressed as a percentage. Enantiomeric excess is defined as 100 x (er - 1) / (er + 1), where "er" is the ratio of the more abundant enantiomer to the less abundant enantiomer.

''Enantioselectivity'' refers to a given reaction (e.g., hydrogenation) that yields more of one enantiomer than another.

"High level of enantioselectivity" refers to a given reaction that yields product with an ee of at least about 80 %.

"Enantiomerically enriched" refers to a sample of a chiral compound, which has more of one enantiomer than another. The degree of enrichment is measured by er or ee.

"Substantially pure enantiomer" or "substantially enantiopure" refers to a sample of an enantiomer having an ee of about 90 % or greater.

"Enantiomerically pure" or "enantiopure" refers to a sample of an enantiomer having an ee of about 99.9 % or greater.

"Opposite enantiomer" refers to a molecule that is a non-superimposable mirror image of a reference molecule, which may be obtained by inverting all of the stereogenic centers of the reference molecule. For example, if the reference molecule has S absolute stereochemical configuration, then the opposite enantiomer has R absolute stereochemical configuration. Likewise, if the reference molecule has S,S absolute stereochemical configuration, then the opposite enantiomer has R,R stereochemical configuration, and so on.

"Pre-catalyst" or "catalyst precursor" refer to a compound or set of compounds that are converted into a catalyst prior to use.

"Pharmaceutically acceptable" refers to substances, which are within the scope of sound medical judgment, suitable for use in contact with the tissues of patients without undue toxicity, irritation, allergic response, and the like, commensurate with a reasonable benefit-to-risk ratio, and effective for their intended use.

"Treating" refers to reversing, alleviating, inhibiting the progress of, or preventing a disorder or condition to which such term applies, or to preventing one or more symptoms of such disorder or condition.

"Treatment" refers to the act of "treating" as defined immediately above.

"About," when used in connection with a measurable numerical variable, refers to the indicated value of the variable and to all values of the variable that are within the experimental error of the indicated value (e.g., within the 95% confidence interval for the mean) or within 10 percent of the indicated value, whichever is greater.

"Solvate" describes a molecular complex comprising pregabalin and a stoichiometric or non-stoichiometric amount of one or more pharmaceutically acceptable solvent molecules (e.g., ethanol).

"Hydrate" describes a solvate comprising a pharmaceutically active ingredient (e.g., pregabalin) and a stoichiometric or non-stoichiometric amount of water.

"Pharmaceutically acceptable esters, amides, and prodrugs" refer to acid or base addition salts, esters, amides, zwitterionic forms, where possible, and prodrugs of claimed and disclosed compounds. Examples of pharmaceutically acceptable, non-toxic esters include, without limitation, C₁₋₆ alkyl esters, C₅₋₇ cycloalkyl esters, and arylalkyl esters of claimed and disclosed compounds, where alkyl, cycloalkyl, and aryl are defined above. Such esters may be prepared by conventional methods, as described, for example, in M.B. Smith and J. March, March's Advanced Organic Chemistry (5th Ed. 2001).

Examples of pharmaceutically acceptable, non-toxic amides include, without limitation, those derived from ammonia, primary C₁₋₆ alkyl amines, and secondary C₁₋₆ dialkyl or heterocyclyl amines of claimed and disclosed compounds, where alkyl and heterocyclyl are defined above. Such amides may be prepared by conventional methods, as described, for example, in *March's Advanced Organic Chemistry.*

"Prodrugs" refer to compounds having little or no pharmacological activity that can, when metabolized in vivo, undergo conversion to claimed or disclosed compounds having desired activity. For a discussion of prodrugs, see T. Higuchi and V Stella, "Pro-drugs as Novel Delivery Systems," ACS Symposium Series 14 (1975), E.B. Roche (ed.), Bioreversible Carriers in Drug Design (1987), and H. Bundgaar, Design of Prodrugs (1985).

Table 1 lists abbreviations used throughout the specification.

**TABLE 1. List of Abbreviations**

| Abbreviation | Description |
|---|---|
| Ac | acetyl |
| ACN | acetonitrile |
| AcNH | acetylamino |
| Aq | aqueous |
| BisP* | (*S,S*)-1,2-bis(*t*-butylinethylphosphino)ethane |
| Bn | benzyl |
| (*R,R*)-Et-BPE | (+)-1,2-bis((2R,5R)-2,5-diethylphospholano)ethane |
| (R,R)-Me-BPE | (+)-1,2-bis((2R,5R)-2,5-dimethylphospholano)ethane |
| Bu | butyl |
| *i*-Bu | isobutyl |
| n-BuLi | normal-butyl lithium |
| Bu₄NBr | tetrabutylammonium bromide |
| t-Bu | tertiary butyl |
| *t*-BuNH₂ | tertiary-butylamine |
| *t*-BuOK | potassium tertiary butyl oxide |
| *t*-BuOMe | tertiary butyl methyl ether |
| *t*-BuONa | sodium tertiary butyl oxide |
| CBz | benzyloxycarbonyl |
| COD | 1,5-cyclooctadiene |
| DABCO | 1,4-diazabicyclo[2.2.2]octane |
| DBU | 1,8-diazabicyclo[5.4.0]undec-7-ene |
| DEAD | diethylazodicarboxylate |
| DIPEA | diisopropylethylamine (Hünig's Base) |
| DMAP | 4-dimethylaminopyridine |
| DMF | dimethylformamide |
| DMSO | dimethylsulfoxide |
| (*R,R*)-Et-DUPHOS | (-)-1,2-bis((2*R*,5*R*)-2,5-diethylphospholano)benzene |
| (*S,S*)-Et-DUPHOS | (-)-1,2-bis((2*S*,5*S*)-2,5-diethylphospholano)benzene |
| (*R,R)*-*i*-Pr-DUPHOS | (+)-1,2-bis((2R,5R)-2,5-di-*i*-propylphospholano)benzene |
| (*R,R*)-Me-DUPHOS | (-)-1,2-bis((2*R*,5*R*)-2,5-dimethylphospholano)benzene |
| (*S,S*)-Me-DUPHOS | (-)-1,2-bis((2*S*,5*S*)-2,5-dimethylphospholano)benzene |
| ee | enantiomeric excess |
| Et | ethyl |
| Et₃N | triethylamine |
| Et₂NH | diethylamine |
| EtOH | ethyl alcohol |
| EtOAc | ethyl acetate |
| h, min, s, d | hours, minutes, seconds, days |
| HOAc | acetic acid |
| IAcOEt | ethyl iodoacetate |
| IPA | isopropanol |
| LiHMDS | lithium hexamethyldisilazide |
| LTMP | lithium tetramethylpiperidide |
| LDA | lithium diisopropylamide |
| Me | methyl |
| MeCl₂ | methylene chloride |
| MeI | methyl iodide |
| MeONa | sodium methoxide |
| MeOH | methyl alcohol |
| Mpa | mega Pascals |
| Ms | mesyl |
| NMP | *N*-methylpyrrolidone |
| OTf⁻ | triflate (trifluoro-methanesulfonic acid anion) |
| Ph | phenyl |
| Ph₃P | triphenylphosphine |
| Ph₃As | triphenylarsine |
| *i*-Pr | isopropyl |
| RI | refractive index |
| RT | room temperature (approximately 20°C-25°C) |
| s/c | substrate-to-catalyst molar ratio |
| Tf | trifluoromethanesulfonyl (triflyl) |
| TFA | trifluoroacetic acid |
| THF | tetrahydrofuran |
| TLC | thin-layer chromatography |
| TMEDA | *N,N,N',N'*-tetramethyl-1,2-ethylenediamine |
| TRITON B | benzyltrimethylammonium hydroxide |
| Ts | tosyl |

In some of the reaction schemes and examples below, certain compounds can be prepared using protecting groups, which prevent undesirable chemical reaction at otherwise reactive sites. Protecting groups may also be used to enhance solubility or otherwise modify physical properties of a compound. For a discussion of protecting group strategies, a description of materials and methods for installing and removing protecting groups, and a compilation of useful protecting groups for common functional groups, including amines, carboxylic acids, alcohols, ketones, aldehydes, and the like, see T. W. Greene and P.G. Wuts, Protecting Groups in Organic Chemistry (1999) and P. Kocienski, Protective Groups (2000).

In addition, some of the schemes and examples below may omit details of common reactions, including oxidations, reductions, and so on, which are known to persons of ordinary skill in the art of organic chemistry. The details of such reactions can be found in a number of treatises, including Richard Larock, Comprehensive Organic Transformations (1999), and the multi-volume series edited by Michael B. Smith and others, Compendium of Organic Synthetic Methods (1974-2003). Generally, starting materials and reagents may be obtained from commercial sources.

The present invention provides materials and methods for preparing chiral compounds represented by Formula 2, above, including pharmaceutically acceptable salts, esters, amides, or prodrugs thereof. In Formula 2, the chiral compounds have at least one stereogenic center, as indicated by the "*", and includes substituents R¹, R², R³, R⁴, and X, which are defined above. Useful compounds represented by Formula 2 include those in which R¹ is amino, amino-C₁₋₆ alkyl, cyano or cyano-C₁₋₆ alkyl; R² is C₁₋₆ alkoxycarbonyl or carboxy; X is -CH₂- or a bond; and R³ and R⁴ are independently hydrogen atom or C₁₋₆ alkyl. Particularly useful compounds include α-, β-, and γ-amino acids in which R¹ is amino or aminomethyl; R² is carboxy; X is a bond or -CH₂-; and R³ and R⁴ are independently hydrogen atom or C₁₋₆ alkyl. Especially useful compounds thus include (*S*)-3-cyano-5-methyl-hexanoic acid, and (*S*)-(+)-3-(aminomethyl)-5-methyl-hexanoic acid, Formula 1, which is known as pregabalin.

Scheme I illustrates a method of preparing a desired enantiomer of the compound of Formula 2. The enantioselective synthesis includes the steps of (a) reacting a prochiral substrate (olefin) of Formula 3, with hydrogen in the presence of a chiral catalyst and organic solvent to yield the compound of Formula 2; and (b) optionally converting the compound of Formula 2 into a pharmaceutically acceptable salt, ester, amide, or prodrug. Substituents R¹, R², R³, R⁴, and X in Formula 3 are as defined in Formula 2.. More generally, and unless stated otherwise, when a particular substituent identifier (R¹, R², R³, etc.) is defined for the first time in connection with a formula, the same substituent identifier, when used in a subsequent formula, will have the same definition as in the earlier formula. Thus, for example, if R²⁰ in a first formula is hydrogen, halogeno, or C₁₋₆ alkyl, then unless stated differently or otherwise clear from the context of the text, R²⁰ in a second formula is also hydrogen, halogeno, or C₁₋₆ alkyl.

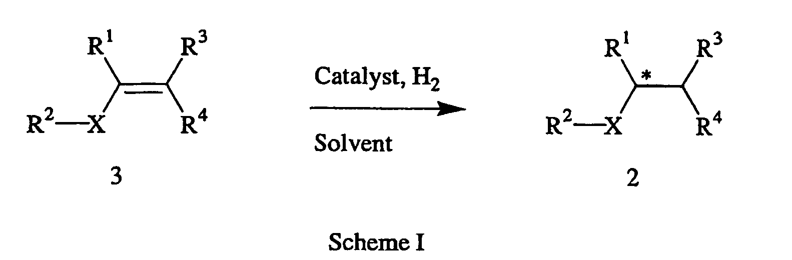

Useful prochiral substrates of Formula 3 include individual Z- or E-isomers or a mixture of Z- and *E-* isomers. Useful prochiral substrates further include compounds of Formula 3 in which R¹ is amino, amino-C₁₋₆ alkyl, cyano or cyano-C₁₋₆ alkyl; R² is C₁₋₆ alkoxycarbonyl, carboxy or -CO₂-Y; X is -CH₂- or a bond; R³ and R⁴ are independently hydrogen atom or C₁₋₆ alkyl; and Y is a cation. Other useful compounds include α-, β-, and γ-cyano acids in which R¹ is cyano or cyanomethyl; R² is carboxy or -CO₂-Y; X is a bond or -CH₂-; R³ and R⁴ are independently hydrogen atom or C₁₋₆ alkyl; and Y is a Group 1 (alkali) metal ion, a Group 2 (alkaline earth) metal ion, a primary ammonium ion, or a secondary ammonium ion. Particularly useful compounds of Formula 3 include 3-cyano-5-methyl-hex-3-ennoic acid or base addition salts thereof, such as 3-cyano-5-methyl-hex-3-enoate t-butyl-ammonium salt. The prochiral substrates may be obtained from commercial sources or may be derived from known methods.

The chiral catalyst comprises a chiral ligand bound to a transition metal (i.e., Group 3-Group 12 metals) through phosphorus atoms, and has a structure represented by Formula 4 or Formula 5 (or its mirror image), as noted above. An especially useful chiral catalyst includes the bisphosphine ligand of Formula 5 having an ee of about 95 % or greater or, preferably, having an ee of about 99 % or greater. Useful transition metals include rhodium, ruthenium, and iridium. Of these, rhodium is especially useful.

The reaction shown in Scheme I may employ a chiral catalyst precursor or pre-catalyst. A catalyst precursor or pre-catalyst is a compound or set of compounds, which are converted into the chiral catalyst prior to use. Catalyst precursors typically comprise a transition metal (e.g., rhodium) complexed with the bisphosphine ligand (e.g., Formula 4) and a diene (e.g., norbornadiene, COD, (2-methylallyl)₂, etc.), a halide (Cl or Br) or a diene and a halide, in the presence of a counterion, A-, such as OTf⁻, PF₆⁻, BF₄⁻, SbF₆⁻, ClO₄⁻, etc. Thus, for example, a catalyst precursor comprised of the complex, [(bisphosphine ligand)Rh(COD)]⁺A⁻ may be converted to a chiral catalyst by hydrogenating the diene (COD) in MeOH to yield [(bisphosphine hgand)Rh(MeOH)₂]⁺A⁻. MeOH is subsequently displaced by the prochiral olefin (Formula 3), which undergoes enantioselective hydrogenation to the desired chiral compound (Formula 2). Thus, for example, a useful chiral catalyst precursor includes (*S*)-(+)-(2-{[(di-*t*-butyl-phosphanyl)-methyl]-methyl-phosphanyl}-2-methyl-propane)-(1,5-cyclooctadiene) rhodium (I) tetrafluroborate

Depending on which enantiomer of the chiral catalyst is used, the asymmetric hydrogenation generates an enantiomeric excess (ee) of an (R)-enantiomer or (S)-enantiomer of Formula 2. Although the amount of the desired enantiomer produced will depend on the reactions conditions (temperature, H₂ pressure, catalyst loading, solvent), an ee of the desired enantiomer of about 80 % or greater is desirable; an ee of about 90 % or greater is more desirable; and an ee of about 95 % is still more desirable. Especially useful asymmetric hydrogenations are those in which the ee of the desired enantiomer is about 99 % or greater. For the purposes of this disclosure, a desired enantiomer of Formula 2 is considered to be substantially pure if it has an ee of about 90 % or greater.

For a given chiral catalyst and prochiral substrate, the molar ratio of the substrate and catalyst (s/c) may depend on, among other things, H₂ pressure, reaction temperature, and solvent. Usually, the substrate-to-catalyst ratio exceeds about 10:1 or 20:1, and substrate-to-catalyst ratios of about 100:1 or 200:1 are common. Although the chiral catalyst may be recycled, higher substrate-to-catalyst ratios are useful. For example, substrate-to-catalyst ratios of about 1000:1, 10,000/1, and 20,000:1, or greater, would be useful. The asymmetric hydrogenation is typically carried out at about RT or above, and under about 0.1 MPa (1 atm) or more of H₂. The temperature of the reaction mixture may range from about 20°C to about 80°C, and the H₂ pressure may range from about 0.1 MPa to about 5 Mpa or higher, but more typically, ranges from about 0.3 Mpa to about 3 Mpa. The combination of temperature, H₂ pressure, and substrate-to-catalyst ratio is generally selected to provide substantially complete conversion (i.e., about 95 wt % or higher) of the prochiral olefin within about 24 h. Generally, increasing the H₂ pressure increases the enantioselectivity.

A variety of organic solvents may be used in the asymmetric hydrogenation, including protic solvents, such as MeOH, EtOH, and *i*-PrOH. Other useful solvents include aprotic polar solvents, such as THF, MeCl₂, and acetone, or aromatic solvents, such as toluene, trifluorotoluene, and chlorobenzene. The enantioselective hydrogenation may employ a single solvent, or may employ a mixture of solvents, such as MeOH and THF.

As shown in Scheme II, the disclosed asymmetric hydrogenation is useful for preparing pregabalin or (*S*)-(+)-3-(aminomethyl)-5-methyl-hexanoic acid (Formula 1). The method may be used to produce pregabalin having an ee of about 95 % or greater, or having an ee of about 99 % or greater, and in some cases having an ee of about 99.9 % or greater. The method includes the enantioselective hydrogenation of the compound of Formula 6 using a chiral catalyst to yield a chiral cyano precursor of pregabalin (Formula 7). The chiral cyano precursor is subsequently reduced and optionally treated with an acid to yield pregabalin. In Formula 6-8, substituent R⁵ can be carboxy group or -CO₂-Y, where Y is a cation.

Useful prochiral substrates (Formula 6) include a base addition salt of 3-cyano-5-methyl-hex-3-enoic acid, such as 3-cyano-5-methyl-hex-3-enoate t-butyl-ammonium salt. Other useful prochiral substrates include those in which Y in Formula 6 is a Group 1 metal ion, a Group 2 metal ion, a primary ammonium ion, or a secondary ammonium ion. The prochiral substrate may be obtained from commercial sources or may be derived from known methods. For a discussion of the preparation of useful prochiral substrates and the reduction of chiral cyano pregabalin precursors, see, for example, commonly assigned U.S. Patent Application No. 2003/0212290 A1, published November 13, 2003.

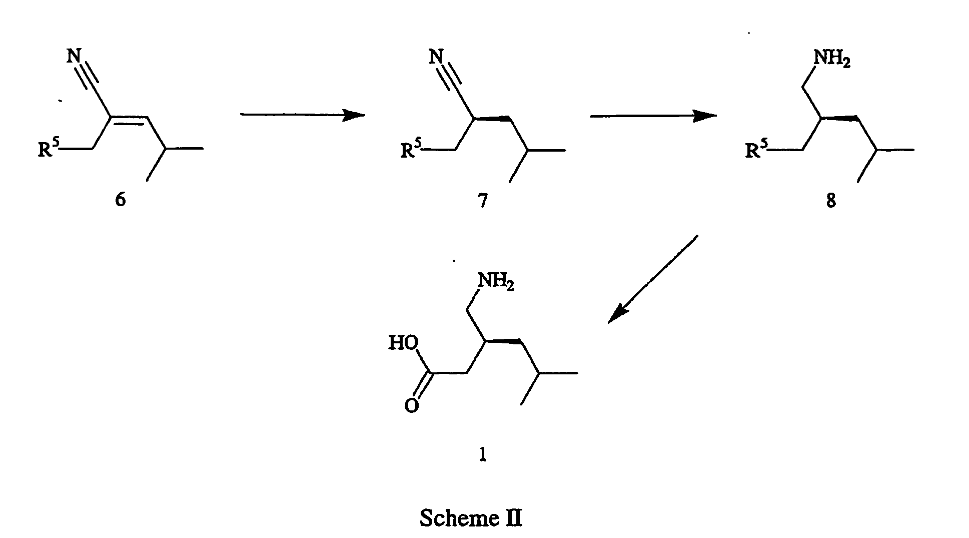

Scheme III shows a method for preparing the chiral ligand of Formula 4. The method may be used to prepare either the *R*-enantiomer (Formula 5) or the S-enantiomer, each having an ee of about 80 %, 90 %, 95 %, or 99 % or greater. As shown in Scheme III, the method includes reacting a first monophosphine (Formula 9) with a second monophosphine (Formula 10) to yield a first bisphosphine intermediate (Formula 11), in which the first monophosphine is treated with a base prior to reaction, X is a leaving group (e.g., halogeno such as chloro), and R⁶ is typically BH₃, but can also be sulfur or oxygen. The method further includes reacting the first bisphosphine intermediate (Formula 11) with a borane or with sulfur or oxygen to yield a second bisphosphine intermediate (Formula 12), in which R⁷ is the same as or different than R⁶ and is BH₃, sulfur, or oxygen. Substituents R⁶ and R⁷ are subsequently removed to yield the chiral bisphosphine ligand of Formula 4. Though not shown in Scheme III, the second bisphosphine intermediate (Formula 12) is resolved into separate enantiomers before or after removal of R⁶ and R⁷.

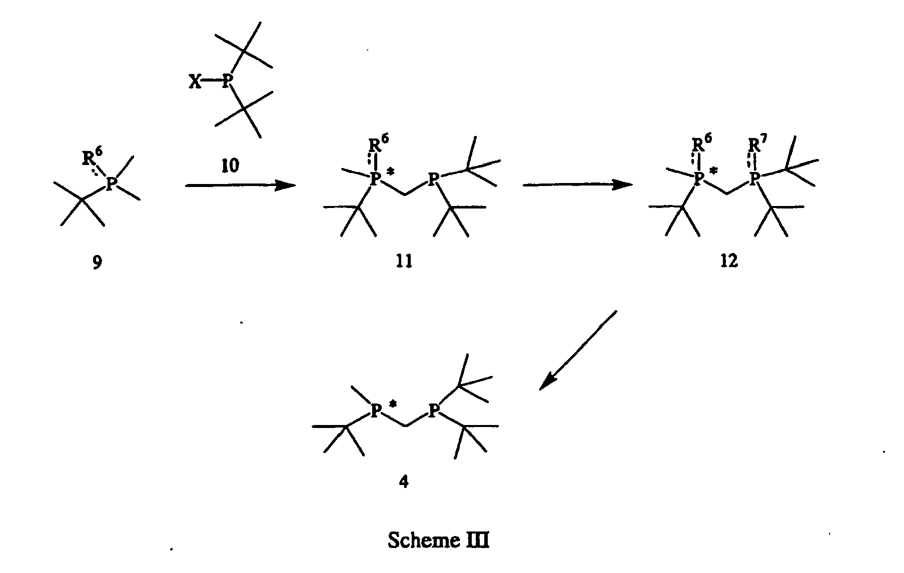

Substituents R⁶ and R⁷ may be removed many different ways depending on the nature of the particular substituents. For instance, when R⁶ and R⁷ are each BH₃ (Formula 13), they may be removed by reacting the second bisphosphine intermediate with an amine or an acid to yield the compound of Formula 4. Thus, for example, the compound of Formula 13 may be reacted with BBF₄**•**Me₂O, followed by base hydrolysis to yield the compound of Formula 4. Similarly, the compound of Formula 13 may be treated with DABCO, TMEDA, DBU, or Et₂NH, or combinations thereof to remove R⁶ and R⁷. See, for example, H. Bisset et al., Tetrahedon Letters 34(28):4523-26 (1993); see also, commonly assigned U.S. Patent Application No. 2003/0143214 A1, published October 3, 2002, and commonly assigned U.S. Patent Application No. 2003/0073868.

When both substituents are sulfur atoms (Formula 14), R⁶ and R⁷ may be removed using techniques shown in Scheme IV. One of the methods includes the steps of (a) reacting the compound of Formula 14 with R⁸OTf to yield a compound of Formula 15, in which R⁸ is a C₁₋₄ alkyl (e.g., methyl); (b) reacting the compound of Formula 15 with a borohydride (e.g., LiBH₄) to yield the compound of Formula 13; and (c) reacting the compound of Formula 13 with an amine or an acid to yield the compound of Formula 4. Another method includes steps (a) and (b) above, and further includes the steps of (c) reacting the compound of Formula 13 with HCl, which is dispersed in a polar aprotic solvent, to yield a compound of Formula 15, and (d) reacting the compound of Formula 16 with an amine or an acid to yield the compound of Formula 4.

When both substituents are sulfur or oxygen, R⁶ and R⁷ may also be removed by treating the compound of Formula 12 with a reducing agent, including a perchloropolysilane such as hexachlorodisilane. For a discussion of the use of a perchloropolysilane for stereospecific deoxygenation of an acyclic phosphine oxide, see K. Naumann et al., J. Amer. Chem. Soc. 91(25):7012-23 (1969).

As noted above in connection Scheme I, the methods used to convert the prochiral substrates of Formula 3 or Formula 6 to the desired enantiomers of Formula 1 or Formula 7, employ chiral catalysts or catalyst precursors, which are converted to the chiral catalysts prior to use. The catalyst or pre-catalysts are comprised of the chiral ligand of Formula 4 or Formula 5 (or its mirror-image) bound to a transition metal (e.g., Rh) through phosphorus atoms.

The catalyst or pre-catalyst may be prepared using the method shown in Scheme V. The method includes the steps of (a) removing substituents R⁹ to yield a compound of Formula 4, in which R⁹ is BH₃, sulfur, or oxygen; and (b) binding the compound of Formula 4 to a transition metal (e.g., rhodium). Step (b) generally includes reacting the compound of Formula 4 with a complex of Formula 18, in which ligands L¹ and L² are, respectively, a diene or anionic ligand as defined above, A is a negatively-charged counterion as defined above, and m, n, and p are, respectively, an integer from 0 to 2, inclusive, an integer from 0 to 4, inclusive, and a positive odd integer, such that 4xm + 2xn + p = 9. The pre-catalyst may provide certain advantages over either the free ligand (Formula 4) or the chiral catalyst, such as improved stability during storage, ease of handling (e.g., a solid rather than a liquid), and the like.

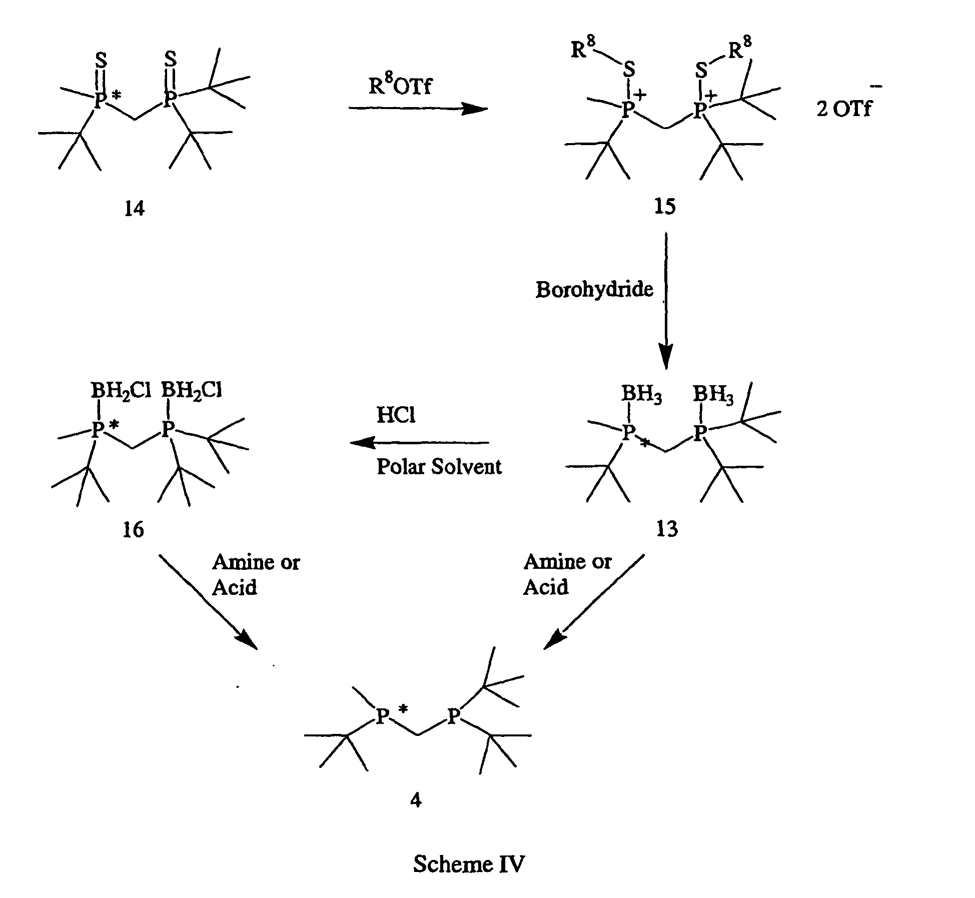

Generally, the chemical transformations described throughout the specification may be carried out using substantially stoichiometric amounts of reactants, though certain reactions may benefit from using an excess of one or more of the reactants. Additionally, many of the reactions disclosed throughout the specification, including the asymmetric hydrogenation of the compounds of Formula 2 and Formula 6, may be carried out at about RT, but particular reactions may require the use of higher or lower temperatures, depending on reaction kinetics, yields, and the like. In addition, any reference in the disclosure to a stoichiometric range, a temperature range, a pH range, etc., includes the indicated endpoints.

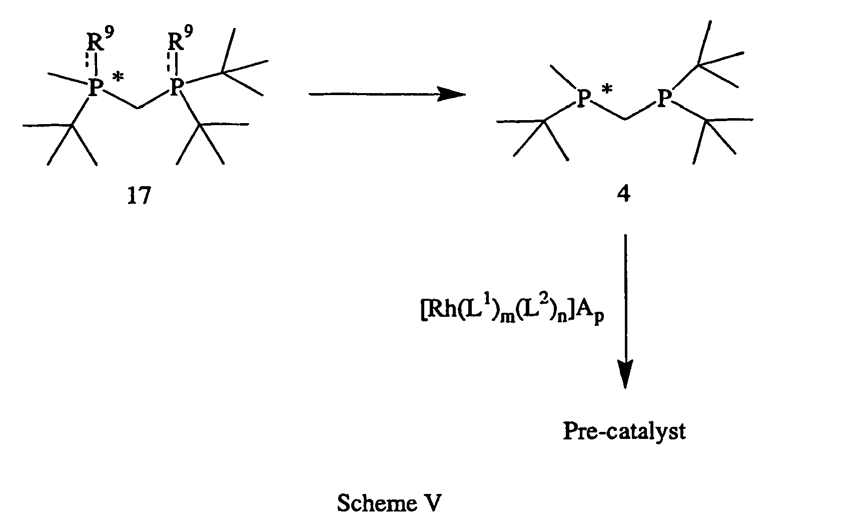

The desired (*S*)- or (R)-enantiomers of any of the compounds disclosed herein may be further enriched through classical resolution, chiral chromatography, or recrystallization. For example, the compounds of Formula 1 or Formula 2 may be reacted with an enantiomerically-pure compound (e.g., acid or base) to yield a pair of diastereoisomers, each composed of a single enantiomer, which are separated via, say, fractional recrystallization or chromatography. The desired enantiomer is subsequently regenerated from the appropriate diastereoisomer. Additionally, the desired enantiomer often may be further enriched by recrystallization in a suitable solvent when it is it available in sufficient quantity (e.g., typically not much less than about 85 % ee, and in some cases, not much less than about 90 % ee).

Many of the compounds described in this disclosure, including those represented by Formula 1, 2, 8, and 32 are capable of forming pharmaceutically acceptable salts. These salts include, without limitation, acid addition salts (including diacids) and base salts. Pharmaceutically acceptable acid addition salts include nontoxic salts derived from inorganic acids such as hydrochloric, nitric, phosphoric, sulfuric, hydrobromic, hydroiodic, hydrofluoric, phosphorous, and the like, as well nontoxic salts derived from organic acids, such as aliphatic mono- and dicarboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, malate, tartrate, methanesulfonate, and the like.

Pharmaceutically acceptable base salts include nontoxic salts derived from bases, including metal cations, such as an alkali or alkaline earth metal cation, as well as amines. Examples of suitable metal cations include, without limitation, sodium cations (Na⁺), potassium cations (K⁺), magnesium cations (Mg²⁺), calcium cations (Ca²⁺), and the like. Examples of suitable amines include, without limitation, *N,N'*-dibenzylethylenediamine, chloroprocaine, choline, diethanolamine, dicyclohexylamine, ethylenediamine, *N*-methylglucamine, and procaine. For a discussion of useful acid addition and base salts, see S. M. Berge et al., "Pharmaceutical Salts," 66 J. of Pharm. Sci., 1-19 (1977); see also Stahl and Wermuth, Handbook of Pharmaceutical Salts: Properties, Selection, and Use (2002).

One may prepare a pharmaceutically acceptable acid addition salt (or base salt) by contacting a compound's free base (or free acid) with a sufficient amount of a desired acid (or base) to produce a nontoxic salt. One may then isolate the salt by filtration if it precipitates from solution, or by evaporation to recover the salt. One may also regenerate the free base (or free acid) by contacting the acid addition salt with a base (or the base salt with an acid). The degree of ionization in the resulting salt may vary from completely ionized to almost non-ionized.

Claimed and disclosed compounds may exist in both unsolvated and solvated forms and as other types of complexes besides salts. Useful complexes include clathrates or drug-host inclusion complexes where the drug and host are present in stoichiometric or non-stoichiometric amounts. Useful complexes may also contain two or more organic, inorganic, or organic and inorganic components in stoichiometric or non-stoichiometric amounts. The resulting complexes may be ionized, partially ionized, or non-ionized. For a review of such complexes, see J. K. Haleblian, J. Pharm. Sci. 64(8):1269-88 (1975).

Useful forms of the claimed and disclosed compounds, including compounds represented by Formula 1, 2, 8 and 32, include all polymorphs and crystal habits, as well as stereoisomers (geometric isomers, enantiomers, and diastereomers), which may be pure, substantially pure, enriched, or racemic. Useful forms of the claimed and disclosed compounds also include tautomeric forms, where possible.

Additionally, certain compounds of this disclosure, including those represented by Formula 1, 2, 8 and 32, may exist as an unsolvated form or as a solvated form, including hydrated forms. Pharmaceutically acceptable solvates include hydrates and solvates in which the crystallization solvent may be isotopically substituted, e.g. D₂O, d₆-acetone, d₆-DMSO, etc. Unless expressly noted, all references to the free base, the free acid, zwitterion, or the unsolvated form of a compound also includes the corresponding acid addition salt, base salt or solvated form of the compound.

The disclosed compounds also include all pharmaceutically acceptable isotopic variations, in which at least one atom is replaced by an atom having the same atomic number, but an atomic mass different from the atomic mass usually found in nature. Examples of isotopes suitable for inclusion in the disclosed compounds include, without limitation, isotopes of hydrogen, such as ²H and ³H; isotopes of carbon, such as ¹³C and ¹⁴C; isotopes of nitrogen, such as ¹⁵N; isotopes of oxygen, such as ¹⁷O and ¹⁸O; isotopes of phosphorus, such as ³¹P and ³²P; isotopes of sulfur, such as ³⁵S; isotopes of fluorine, such as ¹⁸F; and isotopes of chlorine, such as ³⁶Cl. Use of isotopic variations (e.g., deuterium, ²H) may afford certain therapeutic advantages resulting from greater metabolic stability, for example, increased in vivo half-life or reduced dosage requirements. Additionally, certain isotopic variations of the disclosed compounds may incorporate a radioactive isotope (e.g., tritium, ³H, or ¹⁴C), which may be useful in drug and/or substrate tissue distribution studies.

### EXAMPLES

The following examples are intended to be illustrative and non-limiting, and represent specific embodiments of the present invention.

### GENERAL METHODS AND MATERIALS

All reactions and manipulations were performed under nitrogen in standard laboratory glassware. Asymmetric hydrogenation was performed in a nitrogen-filled glovebox. THF (anhydrous, 99.9%), ACN (anhydrous, 99.8%), diethyl ether (anhydrous, 99.8%), MeOH (anhydrous, 99.8%), and MeCl₂ (anhydrous, 99.8%) were obtained from ALDRICH. Bis(1,5-cyclooetadiene)rhodium (I) tetrafluoroborate was synthesized according to a procedure in T. G. Schenk et al., Inorg. Chem. 24:2334 (1985). Hydrogen gas was used from a lecture bottle supplied by SPECIALTY GAS. Hydrogenations were performed in a Griffin-Worden pressure vessel supplied by KIMBLE/KONTES.

### NUCLEAR MAGNETIC RESONANCE

400 MHz ¹H NMR, 100 MHz ¹³C NMR, and 162 MHz ³¹P NMR spectra were obtained on a VARIAN INOVA400 spectrometer equipped with an Auto Switchable 4-Nuclei PFG probe, two RF channels, and a SMS-100 sample changer by ZYMARK. Spectra were generally acquired near RT, and standard autolock, autoshim and autogain routines were employed. Samples were usually spun at 20 Hz for 1D experiments. ¹H NMR spectra were acquired using 45-degree tip angle pulses, 1.0 s recycle delay, and 16 scans at a resolution of 0.25 Hz/point. The acquisition window was typically 8000 Hz from +18 to -2 ppm (Reference TMS at 0 ppm), and processing was with 0.2 Hz line broadening. Typical acquisition time was 80 s. Regular ¹³C NMR spectra were acquired using 45-degree tip angle pulses, 2.0 s recycle delay, and 2048 scans at a resolution of 1 Hz/point. Spectral width was typically 25 KHz from +235 to -15 ppm (Reference TMS at 0 ppm). Proton decoupling was applied continuously, and 2 Hz line broadening was applied during processing. Typical acquisition time was 102 min. ³¹P NMR spectra were acquired using 45-degree tip angle pulses, 1.0 s recycle delay, and 64 scans at a resolution of 2 Hz/point. Spectral width was typically 48 KHz from +200 to -100 ppm (Reference 85% Phosphoric Acid at 0 ppm). Proton decoupling was applied continuously, and 2 Hz line broadening was applied during processing. Typical acquisition time was 1.5 min.

### MASS SPECTROMETRY.

Mass Spectrometry was performed on a MICROMASS Platform LC system operating under MassLynx and OpenLynx open access software. The LC was equipped with an HP1100 quaternary LC system and a GILSON 215 liquid handler as an autosampler. Data were acquired under atmospheric pressure chemical ionization with 80:20 ACN/water as the solvent. Temperatures: probe was 450°C, source was 150°C. Corona discharge was 3500 V for positive ion and 3200 V for negative ion.

### HIGH PERFORMANCE LIQUID CHROMATOGRAPHY

High Performance Liquid Chromatography (HPLC) was performed on a series 1100 AGILENT TECHNOLOGIES instrument equipped with a manual injector, quaternary pump, and a UV detector. The LC was PC controlled using HP Chemstation Plus Software. Normal Phase chiral HPLC was performed using a Chiracel OJ column supplied by CHIRAL TECHNOLOGIES.

### GAS CHROMATOGRAPHY

Gas Chromatography (GC) was performed on a 110 volt VARIAN STAR 3400 equipped with an FID detector with electrometer, a model 1061 packed/530 µm ID flash injector, a model 1077 split/splitless capillary injector, a relay board that monitors four external events, and an inboard printer/plotter. Gas chromatography was performed using 40 m x 0.25 mm CHIRALDEX G-TA or B-TA columns supplied by ADVANCED SEPARATION TECHNOLOGIES, INC. or on a 25 m x 0.25 mm coating CHIRASIL-L-VAL column supplied by CHROMPACK.

### EXAMPLE 1. Preparation of (2-{[(di-t-butyl-phosphanyl)-methyl]-methyl-phosphanyl}-2-methyl-propane)-diborane (Formula 13)

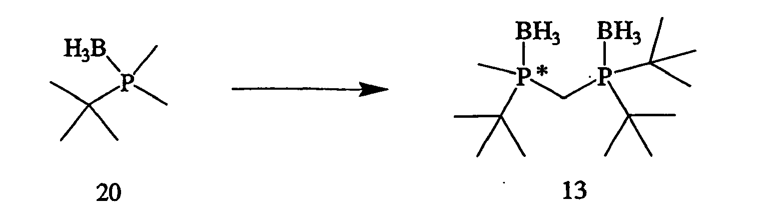

A solution of *t*-butyl-dimethyl-phosphine borane (Formula 20) (20.1 g, 152 mmole) in THF (50 mL) was stirred at 0°C. To the solution was added s-BuLi (104 mL, 145 mmole) over a 20 min period while maintaining the reaction temperature below 20°C. Following the addition, the solution turned slightly cloudy and orange. The reaction was stirred for one hour at 0°C. The solution was subsequently transferred over a 20 min period, via a cannula, to a pre-cooled solution of di-*t-*butylchlorophosphine (25 g, 138 mmole) in THF (50 mL) at 0°C, which turned red immediately upon addition. The temperature was maintained below 20°C during the transfer. Following addition, the reaction was stirred at 0°C for 2 h. To this solution was added BH₃•Me₂S (14.4 mL, 152 mmole) over 10 min while maintaining the reaction temperature below 20°C. The reaction was stirred for 1 h, after which it was poured onto 100 g of ice in IN HCl (100 mL) and stirred for 30 min. The aqueous solution was extracted with EtOAc (2x100 mL) and the combined organic layers were dried over MgSO₄ and filtered. Volatiles were then removed on a rotary evaporator. The residue was recrystallized from hot heptane to yield the titled compound (racemate) as a white crystalline solid. The solid weighed 25 g (63 %); mp = 150-152°C; ¹H NMR (400 MHz, CDCl₃) δ 1.88 (t, J = 12 Hz, 2H), 1.56 (d, J = 10 Hz, 3H), 1.33 (d, J = 13 Hz, 9H), 1.27 (d, J = 13 Hz, 9H), 1.19 (d, J =13 Hz, 9H), 0.61 (br q, 6H).; ¹³C NMR (100 MHz, CDCl₃) δ 34.29 (d, J = 25 Hz), 33.41 (d, J = 25 Hz), 30.00 (d, 25 Hz), 28.30 (s), 27.89 (s), 25.21 (s), 9.12 (dd, J = 21 and 15 Hz), 6.52 (d, J = 32 Hz); ³¹P NMR (162 MHz, CDCl₃) δ 49.70 - 48.15 (m), 33.03 - 31.56 (m). Anal Calc'd for C₁₄H₃₈B₂P₂: C, 57.98; H, 13.21. Found: C, 57.64; H, 13.01.

### EXAMPLE 2. Preparation of (R)-(-)- and (S)-(+)-(2-{[(di-t-butyl-phosphanyl)-methyl]-methyl-phosphanyl}-2-methyl-propane)-diborane (Formula 21 and 22)

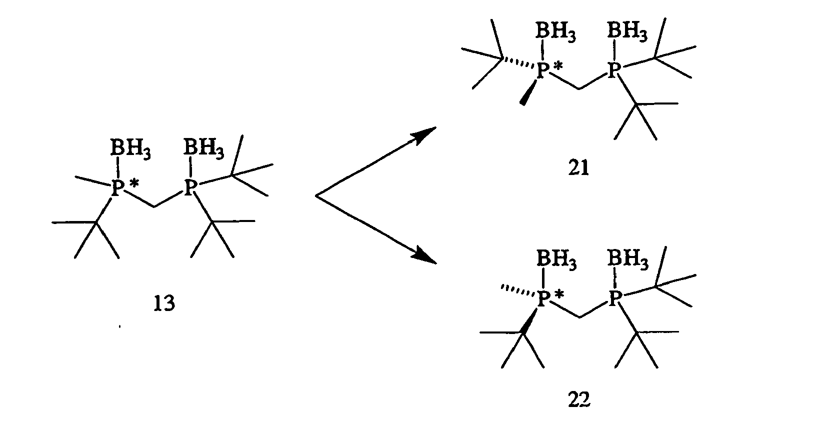

The (*R*)-(-)- and (*S*)-(+)-enantiomers (Formula 21 and 22, respectively) of (2-{[(di-*t*-butyl-phosphanyl)-methyl]-methyl-phosphanyl}-2-methyl-propane)-diborane (Formula 13) were separated by HPLC using a chiral preparatory column and conditions noted in Table 2 below. Since a preparatory-scale RI detector was unavailable, RI detection could not be used to monitor the retention times of the enantiomers. Instead, the solvent was fractionated using a fraction collector and the individual fractions were assayed by HPLC using a chiral analytical column and conditions provided in Table 2. Retention times for the R- and S-enantiomers were 6.8 min, [α]²⁴_{D} = -5.5° (c 0.5, MeOH), and 8.2 min, respectively.

**Table 2. HPLC Conditions for Separating and Analyzing the Enantiomers of (2-{ [(dit-butyl-phosphanyl)-methyl]-methyl-phosphanyl}-2-methyl-propane)-diborane**

| | Preparatory | Analytical |
|---|---|---|
| Column | Daicel Chiralpak AD (250x20 mm, 10 µm) | Daicel Chiralpak AD (250x4.6 mm, 10 µm) |
| Mobile Phase | 99.25:0.75 (hexanes:IPA) | 99.25:0.75 (hexanes:IPA) |
| Flow Rate | 9 mL/min | 1 mL/min |
| Detector | None | RI (35°C) |
| Column Temperature | 30°C | 30°C |
| Concentration | 2 mg/mL | 2 mg/mL |
| Diluent | mobile phase | mobile phase |
| Injection Volume | 500 µL | 25 µL |
| Run Time | 20 min | 13 min |

### EXAMPLE 3. Preparation of (R)-2-{[(di-t-butyl-phosphanyl)-methyl]-methyl-phosphanyl}-2-methyl-propane (Formula 5)

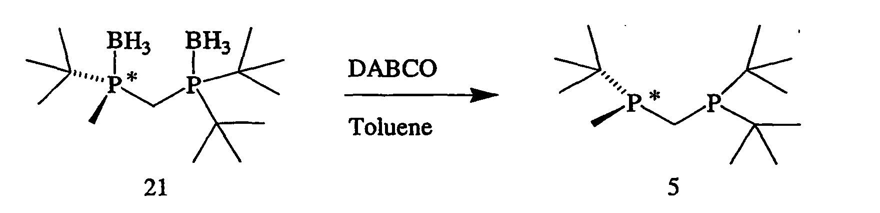

(*R*)-(-)-(2-{[(di-*t*-Butyl-phosphanyl)-methyl]-methyl-phosphanyl}-2-methylpropane)-diborane (Formula 21, 290 mg, 1.0 mmol) and DABCO (135 mg, 1.2 mmol) were dissolved in degassed toluene (10 mL) at 20 °C. The solution was stirred for 4 h at 80°C. The solvent was removed invacuo and the resulting residue was extracted with hexane (3x20 mL). The combined organic extracts were concentrated and dried producing (*R*)-2-{[(di-*t*-butyl-phosphanyl)-methyl]-methyl-phosphanyl}-2-methylpropane (Formula 5, 228 mg, 87 %) as colorless oil. ¹H NMR (400 MHz, CDCl₃) δ 1.47-1.41 (m, 2H), 1.09 (d, J =11 Hz, 9H), 1.03 (d, J = 11 Hz, 9H), 0.94 (d, J =11 Hz, 9H), 0.93 (d, J = 3 Hz, 3H); ¹³C NMR (100 MHz, CDCl₃) δ 7.44 (dd, J = 19 and 6 Hz), 16.09 (dd, J = 32 and 25 Hz), 26.63 (d, J = 14 Hz), 27.95 (dd, J = 23 and 3 Hz), 29.73 (d, J = 14 Hz), 30.16 (dd, J =13 and 4 Hz), 31.70 (dd, J = 23 and 9 Hz), 32.16 (dd, J = 23 and 3 Hz); ³¹P NMR (162 MHz, CDCl₃) δ -13.66 (br m), 18.35 (br m).

### EXAMPLE 4. Preparation of (S)-(+)-(2-{ [(di-t-butyl-phosphanyl)-methyl]-methyl-phosphanyl}-2-methyl-propane)-(1,5-cyclooctadiene) rhodium (I) tetrafluroborate (Formula 23)

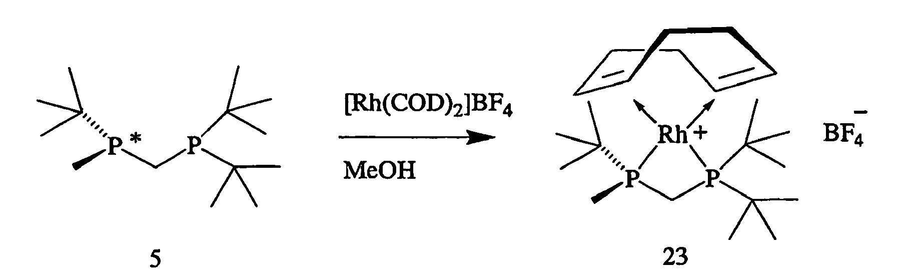

A solution of (*R*)-2-{[(di-*t*-butyl-phosphanyl)-methyl]-methyl-phosphanyl}-2-methyl-propane (Formula 5, 66 mg, 0.25 mmol) in THF (5 mL) was added drop wise to a solution of [Rh(COD)₂]BF₄ (102 mg, 0.25 mmol) in MeOH (10 mL) at 20°C with stirring. After addition, the reaction mixture was stirred for 1 h and solvent was removed invacuo to provide a red solid. Recrystallization of product from warm THF provided (S)-(+)-(2- {[(di-t-butyl-phosphanyl)-methyl]-methyl-phosphanyl}-2-methyl-propane)-(1,5-cyclooctadiene) rhodium (I) tetrafluroborate (Formula 23, 89 mg, 64 %) as a red crystalline product. [α]²⁴_{D} = +52.4° (c 0.9, MeOH); ¹H NMR (400 MHz, CDCl₃) δ 5.63 - 5.51 (m, 2H), 5.11 (br s, 2H), 3.48 - 3.328 (m, 1H), 3.14 (dt, J = 17 and 10 Hz, 1H), 2.49 - 2.25 (m, 4H), 2.21 - 2.09 (m, 4H), 1.69 (d, J = 9 Hz, 3H), 1.39 (d, J = 14 Hz, 9H), 1.33 (d, J = 14 Hz, 9H), 1.13 (d, J = 16 Hz, 9H); ¹³C NMR (100 MHz, CDCl₃) δ 100.20 (dd, J = 9 and 6 Hz), 97.70 (dd, J = 9 and 6 Hz), 92.95 (t, J = 8 Hz), 92.27 (d, J = 8 Hz), 37.68 (m), 36.04 (d, J = 9 Hz), 32.54 (m), 31.48 (s), 30.94 (s), 30.09 (d, J = 5 Hz), 29.81 (d, J = 5 Hz), 29.32 (s), 29.16 (s), 26.57 (d, J = 5 Hz), 9.58 (d, J = 21 Hz); ³¹P NMR (162 MHz, CDCl₃) δ -3.97 (dd, J =126 and 56 Hz), -29.36 (dd, J = 126 and 56 Hz). Anal Calc'd for C₂₁H₄₂B₁F₄P₂Rh₁: C, 46.18; H, 7.75. Found: C, 45.66; H, 7.19.

### EXAMPLES 5-9. Preparation of chiral compounds (Formula 2) via asymmetric hydrogenation of prochiral substrates (Formula 3) using (S)-(+)-(2-{[(di-t-butyl-phosphanyl)-methyl]-methyl-phosphanyl}-2-methyl-propane)-(1,5-cyclooctadiene) rhodium (I) tetrafluroborate (Formula 23).

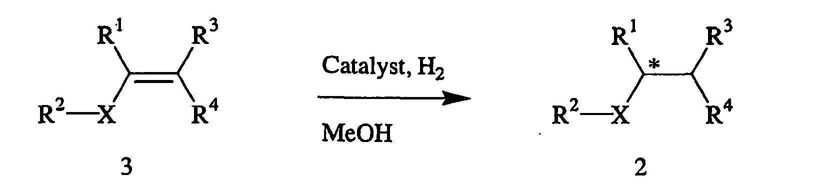

Table 3 lists substrates (Formula 3), ee, and absolute stereochemical configuration of chiral products (Formula 2) prepared via asymmetric hydrogenation using chiral catalyst precursor, (*S*)-(+)-(2-{[(di-*t*-butyl-phosphanyl)-methyl]-methyl-phosphanyl}-2-methyl-propane)-(1,5-cyclooctadiene) rhodium (I) tetrafluroborate (Formula 23). For each entry in Table 3, the catalyst precursor (0.01 mmole) was dissolved in degassed MeOH (1 mL) in a Griffin-Worden pressure vessel equipped with the attachments necessary to connect to a lecture bottle. The substrate (1 mmole) was first dissolved in MeOH (4 mL) and then delivered to the catalyst-MeOH solution via syringe. The vessel was sealed and pressurized to 50 psi H₂. The time to the completion of reaction was determined by the cessation of H₂ gas uptake.

**Table 3. Enantioselectivity of Chiral Compounds (Formula 2) Prepared via Asymmetric Hydrogenation of Prochiral Substrates (Formula 3)**

| Example | R¹ | R² | R³ | R⁴ | X | ee | Config. |
|---|---|---|---|---|---|---|---|
| 5 | AcNH | CO₂H | H | H | Bond | >99 % | R |
| 6 | AcNH | CO₂H | Ph | H | Bond | >99 % | R |
| 7 | AcNH | CO₂Me | H | H | Bond | >99 % | R |
| 8 | AcNH | CO₂Me | Ph | H | Bond | >99 % | R |
| 9 | AcNH | CO₂Me | -C₅H₁₀- | | Bond | 99 % | R |

For each of the reactions shown in Table 3, enantiomeric excess was determined via chiral GC or chiral HPLC. Table 4 provides details of the ee methodology. To determine ee's for *N*-acetylalanine (Example 5) and *N-*acetylphenylalanine (Example 6), each compound was treated with trimethylsilyldiazomethane to convert it to its corresponding methyl ester, which was analyzed as provided in Example 7 or Example 8, respectively. Absolute stereochemical configuration was determined by comparing the signs of optical rotation with those of literature values: (*S*)-*N*-acetylalanine methyl ester [α]²⁰_{D} = -91.7° (*c* 2, H₂O), J. P. Wolf III & C. Neimann, Biochemistry 2:493 (1963); (*S*)-*N-*acetylphenylalanine methyl ester [α]²⁰_{D} = +16.4° (*c* 2, MeOH), B. D. Vineyard et al., J. Am. Chem. Soc. 99:5946 (1997); (*S*)-*N*-acetylcyclohexylglycine methyl ester [α]²⁰_{D} = -4.6° (c = 0.13, EtOH), M. J. Burk et al., J. Am. Chem. Soc. 117:9375 (1995).

**Table 4. Conditions for Determining Enantiomeric Excess**

| | Examples 5 & 7 | Examples 6 & 8 | Example 9 |
|---|---|---|---|
| Method | Capillary GC | HPLC | Capillary GC |
| Column | Chrompack Chiral-L-Val (25 m) | Daicel Chiralcel OJ | Chirasil-L-Val (25 m) |
| Mobile Phase | - | 10 % IPA/hexane | - |
| Flow Rate | - | 1 mL/min | - |
| Column Temp. | 120°C | 30°C | 145°C |
| Concentration | - | 2 mg/mL | - |
| Retention time-R | 10.5 min | 11.6 min | 11.3 min |
| Retention time-S | 11.0 min | 17.7 min | 12.0 min |

### EXAMPLES 10-13. Preparation of a chiral pregabalin precursor (Formula 25) via asymmetric hydrogenation of a prochiral substrate (Formula 24) using (S)-(+)-(2-{[(di-t-butyl-phosphanyl)-methyl]-methyl-phosphanyl}-2-methyl-propane)-(1,5-cyclooctadiene) rhodium (I) tetrafluroborate (Formula 23).

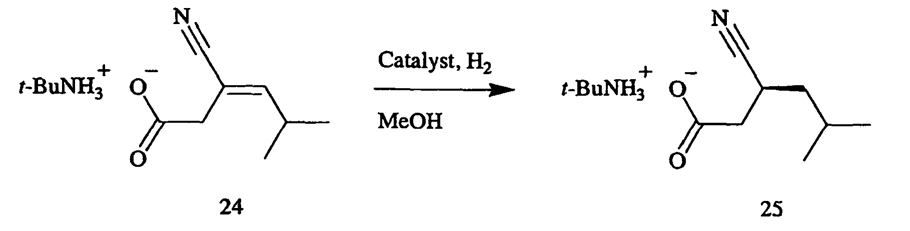

Table 5 lists catalyst (or catalyst precursor), substrate concentration (in MeOH, w/w %), s/c, reaction temperature, H₂ pressure, time to completion, and ee for the preparation of (S)-3-cyano-5-methyl-hexanoic acid *t*-butylammonium salt (Formula 25) via asymmetric hydrogenation of 3-cyano-5-methyl-hex-3-enoic acid *t-*butylammonium salt (Formula 24). For each entry in Table 5, the substrate (Formula 24, 100 g, 442 mmole) was weighed into a hydrogenation bottle in air. The hydrogenation bottle was then transferred to a glovebox ([O₂] < 5 ppm). To the substrate was added degassed MeOH (500 mL) with stirring to dissolve the substrate. The requisite amount of catalyst precursor-either (*S*)-(+)-(2-{[(di-*t*-butyl-phosphanyl)-methyl]-methyl-phosphanyl}-2-methyl-propane)-(1,5-cyclooctadiene) rhodium (I) tetrafluroborate (Formula 23) or (*R*,*R*)-Rh-Me-DuPhos-was added to the substrate solution. The hydrogenation vessel was sealed and pressurized to 50 psi H₂ and stirred vigorously with a TEFLON® coated magnet. The pressure of the reaction was maintained at 50 psi H₂. The time to the completion of reaction was measured by the cessation of H₂ gas uptake.

To determine enantiomeric excess, the chiral pregabalin precursors (Formula 25 and its mirror image) were acidified in-situ with 1 N HCl. The organic components were extracted into MeCl₂. After drying over MgSO₄, the volatiles were removed invacuo. The carboxylic acids were treated with trimethylsilyldiazomethane to convert them to their corresponding methyl esters, which were subsequently analyzed using capillary GC (Astec GTA (30 m), 140°C, isothermal, *R* t₁ = 8.8 min, S t₂ = 9.5 min). Absolute Configurations of the chiral pregabalin precursors were determined by comparing the order of elution to an authenticated sample having S-configuration.

### EXAMPLE 14. Preparation of 2-(dimethyl-phosphinothioyl)-2-methyl-propane (Formula 27)

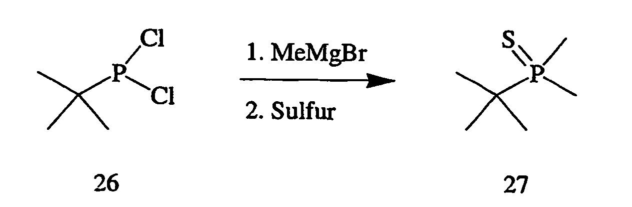

Dichloro-*t*-butyl-phosphine (Formula 26, 10.0 g, 62.9 mmol) was dissolved in THF (100 mL) under N₂ blanket and the resulting solution was cooled to 0°C. MeMgBr (16.5 g, 138 mmol) was added via syringe over a 10 min period. The addition was exothermic. The reaction was warmed to RT and then sulfur (2.22 g, 69.2 mmol) was added in one portion with generation of heat. After stirring for 1 h, the reaction was subjected to a standard aqueous work-up. Recrystallization of the product from heptane yielded 2-(dimethyl-phosphinothioyl)-2-methyl-propane (Formula 27, 8.0 g, 85 % yield).

### EXAMPLE 15. Preparation of 2-[(di-t-butyl-phosphinothioylmethyl)-methyl-phosphinothioyl]-2-methyl-propane (Formula 14)

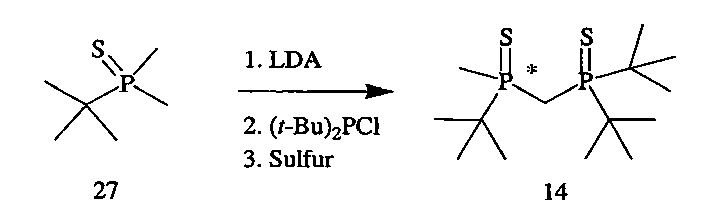

A flask was charged with diisopropylamine (74.2 g, 102.8 mL, mmol) and THF (100 mL) and cooled to -10°C under argon. To the solution was added n-BuLi (44.8 g, 280 mL, 700 mmol) via a dropping funnel while maintaining the temperature below 0°C. To the resulting LDA solution was added, under argon and via a dropping funnel, a solution of 2-(dimethyl-phosphinothioyl)-2-methyl-propane (Formula 27, 50.07 g, 333.3 mmol) dissolved in THF (300 mL). During the addition, the temperature stayed below -5°C. To this solution was added, under argon and via a dropping funnel, a solution of chloro-di-*t*-butylphosphine (60.2 g, 333 mmol) dissolved in THF (80 mL) during which the temperature stayed below -3°C. The reaction mixture was stirred for 1 h at -10°C and was quenched under argon with 6 N HCl (290 mL) while maintaining the temperature below -5°C. After the addition the pH was about 2. Sulfur (11.8 g, 367 mmol) was added in one portion and the reaction mixture was stirred overnight without cooling. The organic layer was separated and then washed with 6 N HCl and then with distilled H₂O. The aqueous layer was extracted with EtOAc. The organic layers were combined and washed with brine, dried over MgSO₄, filtered, and stripped invacuo. The residue was slurried at 40°C in IPA (60 mL) and cooled to 5°C. The solid was collected and washed three times with IPA and then dried invacuo at RT overnight. The procedure yielded 2-[(di-t-butyl-phosphinothioylmethyl)-methyl-phosphinothioyl]-2-methyl-propane (Formula 14) as a white solid (64.6 g, 59 % yield).

### EXAMPLE 16. Preparation of (R)- and (S)-2-[(di-t-butyl-phosphinothioylmethyl)-methyl-phosphinothioyl]-2-methyl-propane (Formula 28 and 29)

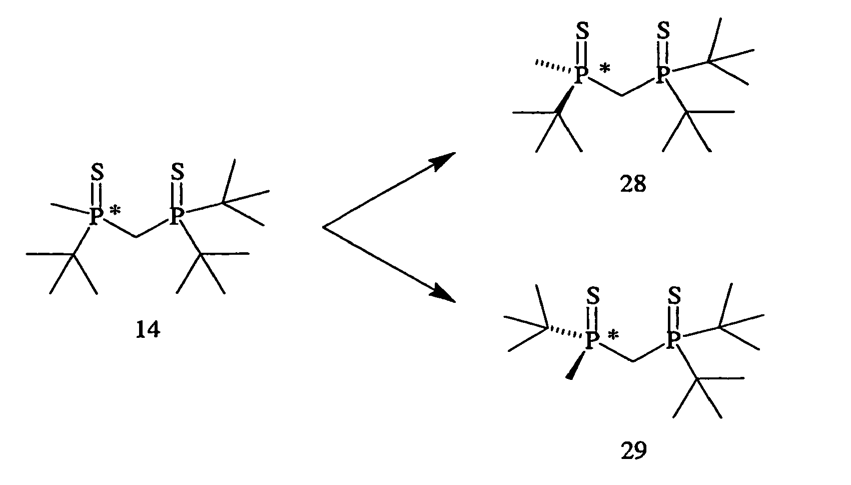

The R- and S-enantiomers (Formula 28 and 29, respectively) of 2-[(di-t-butyl-phosphinothioylmethyl)-methyl-phosphinothioyl]-2-methyl-propane (Formula 14) were separated by HPLC using a chiral preparatory column and conditions noted in Table 5 below. As noted in Table 5, HPLC was also used to check chiral purity and chemical purity.

**Table 5. Separation and the Analysis of the Enantiomers of 2-[(di-t-butyl-phosphinothioylmethyl)-methyl-phosphinothioyl]-2-methyl-propane by HPLC**

| | Preparation | Chiral Purity | Chemical Purity |
|---|---|---|---|
| Column | Daicel Chiralpak AS (250x20 mm, 10 µm) | Daicel Chiralpak AS (250x4.6 mm, 10 µm) | YMC Pack Pro C18 (150x4.6 mm, 3 µm) |
| Mobile Phase A | IPA | IPA | 0.4% HClO₄ (70%) in 9:1 H₂O/MeCN |
| Mobile Phase B | - | - | MeCN |
| Gradient (A) | 100 % | 100 % | 60% to 5% for 15 min 5% to end |
| Equilibration | - | - | 60% A for 8 min |
| Flow Rate | 7.0 mL/min | 0.3 mL/min | 1.0 mL/min |
| Injection Volume | 2 mL | 20 µL | 10 µL |
| Detector | 215 nm | 215 nm | 215 nm |
| Column Temp. | RT | RT | RT |
| Run Time | Stacked injections One every 10 min | 30 min | 33 min w/ equilibration |
| Diluent | IPA | IPA | 1:1 H₂O/MeCN |
| Concentration | 10 mg/mL | 0.3 mg/mL | 0.25 mg/mL |
| Retention time-*R* | | 12.8 min | - |
| Retention time-*S* | | 18.6 min | - |
| Recovery/Purity-*R* | 4.925 g | 100% (Area) | 100% (Area) |
| Recovery/Purity-*S* | 5.241 g | 99.85% (Area) | 99.97% (Area) |

### EXAMPLE 17. Preparation of (S)-(di-t-butyl-methylthio-phosphoniumyl-methyl)-t-butyl-methyl-methylthio-phosphonium di-triflate (Formula 30)

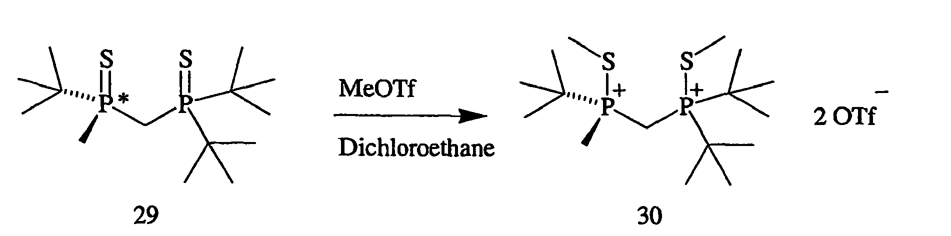

(*S*)-2-[(di-*t*-butyl-phosphinothioylmethyl)-methyl-phosphinothioyl]-2-methylpropane (Formula 29, 5.10 g, 15.6 mmol) was dissolved in 1,2-dichloroethane (50 mL). Methyl triflate (7.69 g, 46.9 mmol)was added to the solution. The reaction mixture was blanketed under argon and stirred at RT. After 10 min MS showed only mono-methylated product. The reaction was stirred overnight whereupon a precipitate had formed (di-methylated product). The solid was collected, washed three times with 1,2-dichloroethane and dried in a vacuum oven at RT to yield, after drying, (*S*)-(di-*t*-butyl-methylthio-phosphoniumyl-methyl)-*t*-butyl-methyl-methylthio-phosphonium di-triflate (Formula 30) as a white solid (6.90 g, 67 % yield).

### EXAMPLE 18. Preparation of (R)-(2-{[(di-t-butyl-phosphanyl)-methyl]-methyl-phosphanyl}-2-methyl-propane)-diborane (Formula 21)

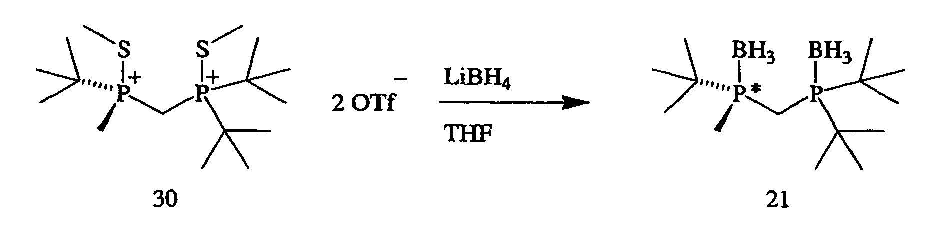

(*S*)-(Di-*t*-butyl-methylthio-phosphoniumyl-methyl)-*t*-butyl-methyl-methylthio-phosphonium di-triflate (2.005 g, 3.063 mmol) was slurried in THF (25 mL). Using an ice bath, the reaction mixture was cooled to 0°C under argon. LiBH₄ (0.400 g, 18.4 mmol) was added via dropping funnel while maintaining the temperature below 5°C. Gas evolution was observed during the addition. After the addition, the ice bath was removed and the reaction was stirred overnight at RT. ¹H NMR showed that some starting material remained. Additional LiBH₄ (3 mL) was added. No gas evolution or exotherm was observed. The reaction mixture was stirred overnight whereupon it was deemed complete via ¹H NMR. The reaction solution was cooled in an ice bath and quenched with 1 N HCl (15 mL). Vigorous evolution of gas was observed. EtOAc was added with stirring. The organic layer was separated and washed with 1 N HCl and H₂O. The aqueous layer was extracted with EtOAc. The combined organic layers were washed with brine, dried over MgSO₄, filtered, and removed invacuo to yield (*R*)-(2-{[(di-*t*-butyl-phosphanyl)-methyl]-methyl-phosphanyl}-2-methyl-propane)-diborane (Formula 21, 0.492 g, 55 % yield). Enantiomeric excess was determined using the analytical procedure described in Table 2, above: ee ≥ 98.7 %; mp = 150-152°C; Anal Calc'd for C₁₄H₃₈B₂P₂: C, 57.98; H, 13.21. Found: C, 57.64; H, 13.01.

### EXAMPLE 19. Preparation of (R)-(2-{[(di-t-butyl-phosphanyl)-methyl]-methyl-phosphanyl}-2-methyl-propane)-di-(chloroborane) (Formula 31)

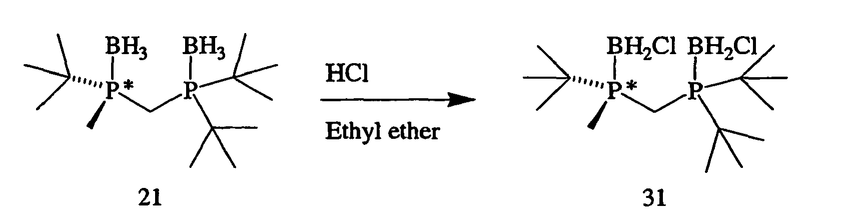

(*R*)-(2-{ [(Di-*t*-butyl-phosphanyl)-methyl]-methyl-phosphanyl }-2-methylpropane)-diborane (Formula 21, 0.200g, 0.690 mmol) was placed in a thick-walled tube equipped with a #15 ACE thread. To the tube was added 2M HCl (0.438 g, 12 mmol) dispersed in ethyl ether (6 mL). Argon was blown over the headspace and the tube was sealed with a #15 ACE plug equipped with a TEFLON® gasket. The contents of the tube were heated to 85°C for 12 h and then cooled to RT, yielding (R)-(2- [(di-*t*-butyl-phosphanyl)-methyl]-methyl-phosphanyl} -2-methyl-propane)-di-(chloroborane) (Formula 31) as a white solid. Since the reaction evolves H₂ gas, care was taken to prevent over pressurization of the tube during and after reaction. The solvent was removed via pipette and the solids were triturated with ethyl ether three times. The solids were dried under vacuum to yield a white solid product (0.222 g, 90 % yield). Because the titled compound is hygroscopic, contact with air was avoided, and the product was stored under vacuum or in a glovebox until use.

### EXAMPLE 20. Preparation of (S)-(+)-(2-{ [(di-t-butyl-phosphanyl)-methyl]-methyl-phosphanyl}-2-methyl-propane)-(1,5-cyclooctadiene) rhodium (I) tetrafluroborate (Formula 23)

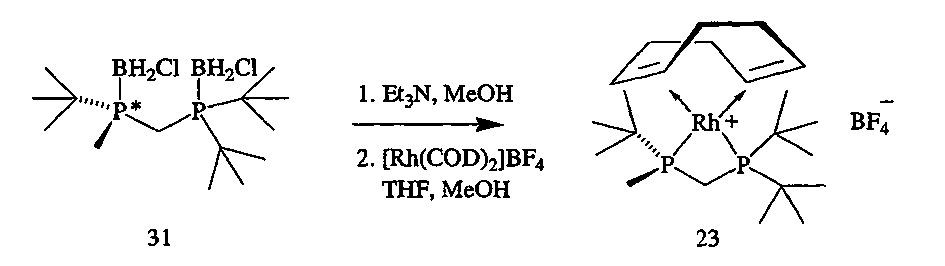

(R)-(2-{ [(Di-*t*-butyl-phosphanyl)-methyl]-methyl-phosphanyl} -2-methyl-propane)-di-(chloroborane) (Formula 31, 179 mg, 0.5 mmol) was dissolved in MeOH (5 mL) and cooled to 0°C. To this solution was added drop wise Et₃N (505 mg, 5.0 mmol). After addition, the mixture was warmed to 20°C and stirred for 30 min. MeOH was removed invacuo and the residue extracted with hexane (3x20 mL). The organic layers were combined, filtered, and concentrated to produce (*R*)*-*2*-*{ [(di-*t-*butyl-phosphanyl)-methyl]-methyl-phosphanyl}-2-methyl-propane (Formula 5, 66 mg). ³¹P & ¹H NMR showed small impurity peaks. The chiral ligand (Formula 5) was dissolved in THF (5 mL) and added drop wise to a solution of [Rh(COD)₂]BF₄ (102 mg, 0.25 mmol) in MeOH (10 mL) at RT with stirring. After addition, the reaction mixture was stirred for 1 h. Solvent was removed invacuo to provide a red solid. Recrystallization of the solid from warm THF provided a red crystalline product. The crystals were washed with 5:1 hexane/diethyl ether and dried invacuo to produce (S)-(+)-(2-{ [(di-*t*-butyl-phosphanyl)-methyl]-methyl-phosphanyl }-2-methylpropane)-(1,5-cyclooctadiene) rhodium (I) tetrafluroborate (Formula 23, 89 mg, 48 % yield from 31). [α]²⁴_{D} = +52.4° (c 0.9, MeOH); Anal Calc'd for C₂₁H₄₂B₁F₄P₂Rh₁: C, 46.18; H, 7.75. Found: C, 45.66; H, 7.19.

### EXAMPLE 21. Preparation of (R)-2-{ [(di-t-butyl-phosphanyl)-methyl]-methyl-phosphanyl}-2-methyl-propane (Formula 5)

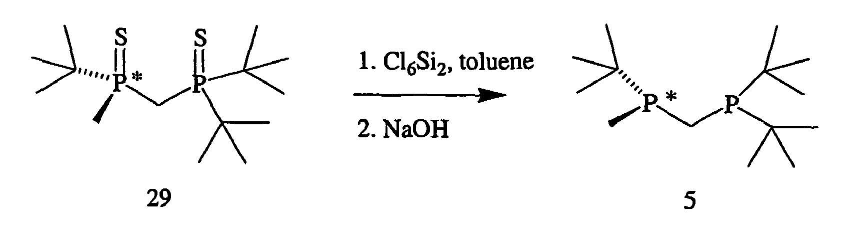

Hexachlorodisilane (2.0 g, 7.5 mmol) was added via syringe to a solution of (*S*)-2-[(di-*t*-butyl-phosphinothioylmethyl)-methyl-phosphinothioyl]-2-methyl-propane (Formula 29, 0.5 g, 1.5 mmol) in degassed toluene (5 mL). The solution was heated with stirring at 80°C for 3 h after which a yellow precipitate had formed. The mixture was then cooled to 0°C and quenched by slowly adding 6.5 N NaOH aq (8 mL) with stirring while maintaining the temperature of the reaction below 70°C. After NaOH addition, the mixture was stirred for 1 h at 50°C until the reaction mixture turned clear. The organic phase was separated in a separatory funnel and the aqueous phase was washed with diethyl ether (2x15 mL). The organic layers were combined and dried over MgSO₄, filtered, and concentrated invacuo to provide (R)-2-{ [(di-t-butyl-phosphanyl)-methyl]-methyl-phosphanyl}-2-methyl-propane (Formula 5) as a colorless oil (0.25 g, 64 % yield). The free phosphine was used directly in the rhodium catalyst formation step (Example 22) without further purification. The preparation of the free phosphine (Formula 5) has been scaled up to 2.2 g of starting material (Formula 29), 5.0 g of starting material, and 10.0 g of starting material, resulting in yields of 82 %, 80 %, and 98 %, respectively.

### EXAMPLE 22. Preparation of (S)-(+)-(2-{[(di-t-butyl-phosphanyl)-methyl]-methyl-phosphanyl}-2-methyl-propane)-(1,5-cyclooctadiene) rhodium (I) tetrafluroborate (Formula 23)

A solution of (R)-2-{[(di-t-butyl-phosphanyl)-methyl]-methyl-phosphanyl}-2-methyl-propane (Formula 5, 0.32 g, 1.2 mmol) in degassed THF (5 mL) was added drop wise at a rate of 1 mL/min and at RT to a solution of [Rh(COD)₂]BF₄ (0.49 g, 1.2 mmol) in degassed MeOH (10 mL) with stirring. The color changed from brown to red. After the addition, the mixture was stirred for 1 h and was concentrated invacuo. The residue was stirred with degassed THF (5 mL) and then filtered. The filtrate was washed with 1:1 diethyl ether/THF (2x5 mL) and then dried invacuo producing an orange dusty solid, (*S*)-(+)-(2-{[(di-*t*-butyl-phosphanyl)-methyl]-methyl-phosphanyl}-2-methyl-propane)-(1,5-cyclooctadiene) rhodium (I) tetrafluroborate (Formula 23, 0.5 g, 75 % yield). The preparation of rhodium complex (Formula 23) has been scaled up to 1.51 g of starting material (Formula 5), 3.27 g of starting material, and 8.15 g of starting material, resulting in yields of 87 %, 92 %, and 91 %, respectively.

### EXAMPLES 23-46. Preparation of chiral compounds (Formula 32) via asymmetric hydrogenation of prochiral olefins (Formula 33) using (S)-(+)-(2-{ [(di-t-butyl-phosphanyl)-methyl]-methyl-phosphanyl }-2-methyl-propane)-(1,5-cyclooctadiene) rhodium (I) tetrafluroborate (Formula 23).

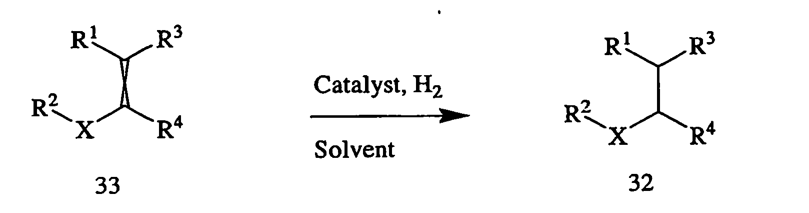

Table 6 lists substrates (Formula 33) and their double bond stereochemical configuration, hydrogen pressure, solvent, ee, and absolute stereochemical configuration of chiral products (Formula 32) prepared via asymmetric hydrogenation using chiral catalyst precursor, (S)-(+)-(2-{ [(di-*t*-butyl-phosphanyl)-methyl]-methyl-phosphanyl}-2-methyl-propane)-(1,5-cyclooctadiene) rhodium (I) tetrafluroborate (Formula 23). For each of the entries in Table 6, the catalyst precursor (Examples 23-45,0.005 mmol; Example 46, 0.025 mmol) and substrate (0.50 mmol, 0.2 M) were dissolved in solvent (2.5 mL) in a Griffin-Worden pressure vessel, which was sealed and pressurized to the desired pressure of H₂. The mixture was vigorously stirred with a PTFE coated magnet at 25°C until H₂ uptake ceased (less than 15 min for Examples 23-45; 6 h for Example 46, as indicated by capillary GC). The H₂ pressure in the bottle was subsequently released, and the reaction mixture was analyzed via chiral GC to provide the percent conversion to product and enantiomeric excess.

**Table 6. Enantioselectivity of Chiral Compounds (Formula 32, R¹=AcNH, X=Bond) Prepared via Asymmetric Hydrogenation of Prochiral Olefins (Formula 33, R¹=AcNH, X=Bond)**

| Example | R² | R³ | R⁴ | Olefin Config. | Solvent | H₂ psi | ee, % Config. |
|---|---|---|---|---|---|---|---|
| 23 | CO₂H | Me | H | E | MeOH | 20 | 99 (R) |
| 24 | CO₂H | Me | H | E | THF | 20 | 99 (R) |
| 25 | CO₂H | Me | H | E | EtOAc | 20 | 99 (R) |
| 26 | CO₂H | Me | H | E | CH₂Cl₂ | 20 | 99 (R) |
| 27 | CO₂H | Me | H | Z | MeOH | 20 | 96 (R) |
| 28 | CO₂H | Me | H | Z | THF | 20 | 96 (R) |
| 29 | CO₂H | Me | H | Z | EtOAc | 20 | 98 (R) |
| 30 | CO₂H | Me | H | Z | CH₂Cl₂ | 20 | 97 (R) |
| 31 | CO₂H | Me | H | Z | THF | 50 | 94 *(R)* |
| 32 | CO₂H | Me | H | Z | THF | 6 | 99 *(R)* |
| 33 | CO₂H | Me | H | *E*/*Z* (1:1) | THF | 20 | 98 *(R)* |
| 34 | CO₂Et | Pr | H | E | THF | 20 | 99 *(R)* |
| 35 | CO₂Et | Pr | H | Z | THF | 20 | 96 *(R)* |
| 36 | CO₂Et | *i*-Bu | H | E | THF | 20 | 98 *(R)* |
| 37 | CO₂Et | *i*-Bu | H. | Z | THF | 20 | 98 *(R)* |
| 38 | CO₂Me | *t*-Bu | H | E | THF | 20 | 99 *(S)* |
| 39 | CO₂Et | Ph | H | Z | THF | 20 | 96 *(S)* |
| 40 | CO₂Et | *i*-Pr | H | E | THF | 20 | 99 (*S*) |
| 41 | CO₂Et | *i*-Pr | H | Z | THF | 20 | 78 (*S*) |
| 42 | CO₂Et | *i*-Pr | H | Z | MeOH | 20 | 69 (*S*) |
| 43 | CO₂Et | *i*-Pr | H | Z | EtOAc | 20 | 84 *(S)* |
| 44 | CO₂Et | *i*-PrH | H | Z | EtOAc | 50 | 66 (*S*) |
| 45 | CO₂Et | *i*-PrH | H | Z | EtOAc | 6 | 92 (*S*) |
| 46 | CO₂Et | -C₃H₆- | | Z | THF | 50 | 85 *(1S,2R)* |

Each of the Z- and E- β-acetamido-β-substituted acrylates (Formula 33) was obtained from an appropriate β-keto ester. A solution of the requisite β-keto ester (24 mmol) and NH₄OAc (9.2 g, 120 mmol) in MeOH (30 mL) was stirred at 20°C for 3 d. After evaporating the solvent, chloroform (50 mL) was added to the residue to give a white solid, which was filtered and washed with chloroform (2 x 50 mL). The combined filtrate was washed with water and brine, and dried over sodium sulfate. Evaporating the solvent provided a β-amino-β-substituted acrylate. To a solution of the β-amino-β-substituted acrylate in THF (24 mL) was added pyridine (12 mL) and anhydrous acetic anhydride (36 mL). The mixture was refluxed for 18 h. The mixture was subsequently cooled to RT and the volatiles were evaporated. The resulting residue was dissolved in EtOAc (40 mL) to give a solution, which was washed with water (20 mL), 1 N HCl (20 mL), 1 M KH₂PO₄ (20 mL), saturated NaHCO₃ (20 mL), and brine (30 mL). The solution was dried over sodium sulfate and residual solvent was evaporated under reduced pressure. Fast chromatography on silica gel with 5:1 and 3:1 hexane/ethyl acetate mobile phases, respectively, provided Z- and E-isomers of the β-acetamido-β-substituted acrylate, which were confirmed by comparison of ¹H NMR data.

Table 7 provides details of the methodology used to determine the stereochemical configuration of products from the reactions shown in Table 6. Enantiomeric excess (ee) was determined via chiral GC using a helium carrier gas at 20 psi. In Table 7, "Column A" refers to CP Chirasil-Dex CB (30 m x 0.25 mm) and "Column B" refers to ChiralDex-gamma-TA (25 m x 0.25 mm). Racemic products were prepared by hydrogenation of corresponding enamines catalyzed by 10% Pd/C in MeOH under 50 psi of H₂ at RT for 2h.

Absolute stereochemical configurations were determined by comparing the signs of optical rotation with literature values given in G. Zhu et al., J. Org. Chem. 64:6907-10 (1999): methyl 3-acetamidobutanoate, [α]_{D}²⁰ = + 8.09 (c 1.24, MeOH), lit. + 21.4 (c 1.4, CHCl₃); ethyl 3-acetamidohexanoate, [α]_{D}²⁰ = + 18.26 (c 1.02, MeOH), lit., ethyl ester, + 42.8 (c 1.86, CHCl₃); ethyl 3-acetamido-4-methypentanoate, [α]_{D}²⁰= + 9.05 (c 1.15, MeOH), lit., ethyl ester, + 52.8 (c 1.2, CHCl₃); ethyl 3-acetamido-5-methylhexanoate, [α]_{D}²⁰ = + 24.44 (c 0.95, MeOH), lit. + 44.6 (c 1.56, CHCl₃); methyl 3-acetamido-4,4-dimethylpentanoate, [α]_{D}²⁰= + 4.86 (c 0.93, MeOH), lit. no report; ethyl 3-acetamido-3-phenylpropanoate, [α]_{D}²⁰= - 47.66 (c 0.91, MeOH), lit. -40.5 (c 2.15, MeOH).

**Table 7. Conditions for Determining Enantiomeric Excess via Chiral GC**

| Examples | 23-33 | 34-35 | 36-37 | 38 | 39 | 40-45 |
|---|---|---|---|---|---|---|
| Column | A | A | A | B | A | A |
| Column Temp., °C | 125 | 108 | 115 | 135 | 145 | 125 |
| Retention time-S, min | 7.67 | 43.86 | 67.01 | 9.78 | 47.64 | 14.89 |
| Retention time-R, min | 8.21 | 44.97 | 69.07 | 9.19 | 45.55 | 14.32 |

It should be noted that, as used in this specification and the appended claims, singular articles such as "a," "an," and "the," may refer to one object or to a plurality of objects unless the context clearly indicates otherwise. Thus, for example, reference to a composition containing "a compound" may include a single compound or two or more compounds.

It is to be understood that the above description is intended to be illustrative and not restrictive. Many embodiments will be apparent to those of skill in the art upon reading the above description. The scope of the invention should, therefore, be determined not with reference to the above description, but should instead be determined with reference to the appended claims, along with the full scope of equivalents to which such claims are entitled. references, including patents, patent applications and publications, are incorporated herein by reference in their entirety and for all purposes.

## Claims

1. A method of making a desired enantiomer of a compound of Formula 2, 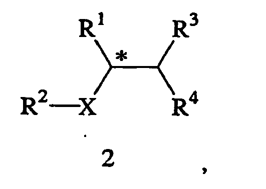 or a pharmaceutically acceptable complex, salt, solvate or hydrate thereof, in which
R¹ is C₁₋₆ alkyl, C₁₋₇ alkanoylamino, C₁₋₆ alkoxycarbonyl, C₁₋₆ alkoxycarbonylamino, amino, amino-C₁₋₆ alkyl, C₁₋₆ alkylamino, cyano, cyano-C₁₋₆ alkyl, carboxy, or -CO₂-Y;
R² is C₁₋₇ alkanoyl, C₁₋₆ alkoxycarbonyl, carboxy, or -CO₂-Y;
R³ and R⁴ are independently hydrogen atom, C₁₋₆ alkyl, C₃₋₇ cycloalkyl, aryl, aryl-C₁₋₆ alkyl, or R³ and R⁴ together are C₂₋₆ alkanediyl;
X is NH-, -O-, -CH₂-, or a bond; and
Y is a cation;
the method comprising:
reacting a compound of Formula 3, 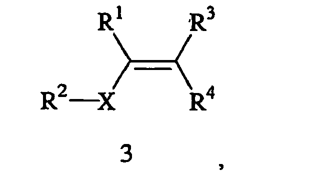 with hydrogen in the presence of a chiral catalyst to yield the compound of Formula 2; and
optionally converting the compound of Formula 2 into a pharmaceutically acceptable salt, complex, solvate or hydrate;
wherein the chiral catalyst comprises a chiral ligand bound to a transition metal through phosphorus atoms, the chiral ligand having a structure represented by Formula 4, 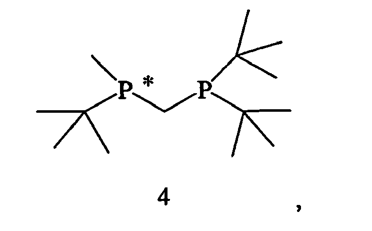 and wherein R¹, R², R³, R⁴, and X in Formula 3 are as defined in Formula 2.

2. A method of making a compound of Formula 1, 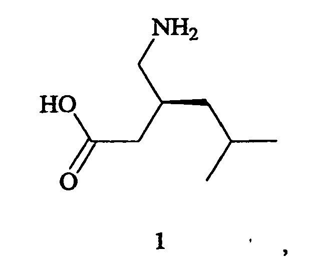 or a pharmaceutically acceptable complex, salt, solvate or hydrate thereof, the method comprising:
reacting a compound of Formula 6, 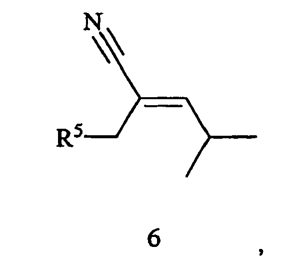 a corresponding Z-isomer of the compound of Formula 6, or a mixture thereof, with hydrogen in the presence of a chiral catalyst to yield a compound of Formula 7, 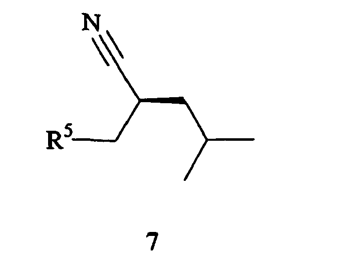
wherein R⁵ is a carboxy group or -CO₂-Y, Y is a cation, and the chiral catalyst comprises a chiral ligand bound to a transition metal through phosphorus atoms, the chiral ligand having a structure represented by Formula 4, 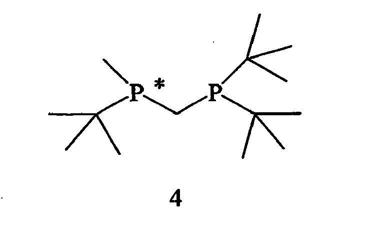 reducing a cyano moiety of the compound of Formula 7 to yield a compound of Formula 8, 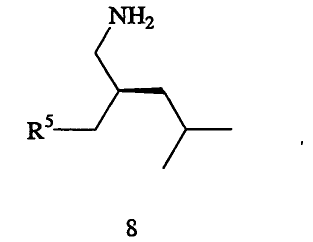 optionally treating the compound of Formula 8 with an acid to yield the compound of Formula 1; and
optionally converting the compound of Formula 8 or Formula 1 to a pharmaceutically acceptable complex, salt, solvate or hydrate.

3. The method of claim 2, wherein the compound of Formula 6 is a base addition salt of 3-cyano-5-methyl-hex-3-enoic acid.

4. The method of claim 3, wherein the compound of Formula 6 is 3-cyano-5-methyl-hex-3-enoate t-butyl-ammonium salt.

5. A method of making a catalyst or pre-catalyst comprised of a chiral ligand bound to a transition metal through phosphorus atoms, the chiral ligand having a structure represented by Formula 4, 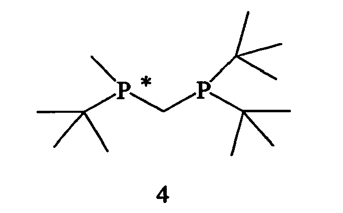 the method comprising:
removing substituent R⁹ from a compound of Formula 17, 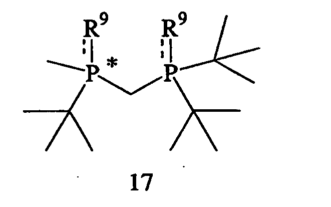 to yield a compound of Formula 4, wherein R⁹ is BH₃, sulfur, or oxygen; and
binding the compound of Formula 4 to a transition metal.

6. A catalyst or pre-catalyst comprising a chiral ligand bound to a transition metal through phosphorus atoms, the chiral ligand having a structure represented by Formula 4,

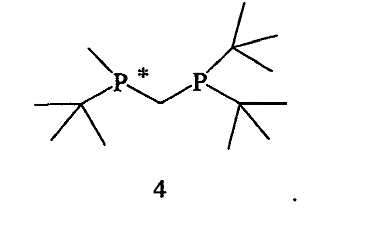

7. A method of making a desired enantiomer of a compound of Formula 32, 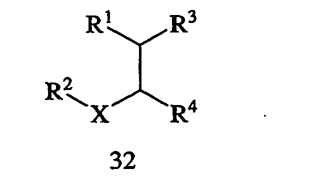 or a pharmaceutically acceptable complex, salt, solvate or hydrate thereof, in which
R¹ is C₁₋₆ alkyl, C₁₋₇ alkanoylamino, C₁₋₆ alkoxycarbonyl, C₁₋₆ alkoxycarbonylamino, amino, amino-C₁₋₆ alkyl, C₁₋₆ alkylamino, cyano, cyano-C₁₋₆ alkyl, carboxy, or -CO₂-Y;
R² is C₁₋₇ alkanoyl, C₁₋₆ alkoxycarbonyl, carboxy, or-CO₂-Y;
R³ and R⁴ are independently hydrogen atom, C₁₋₆ alkyl, C₃₋₇ cycloalkyl, aryl, aryl-C₁₋₆ alkyl, or R³ and R⁴ together are C₂₋₆ alkanediyl;
X is -NH-, -O-, -CH₂-, or a bond; and
Y is a cation;
the method comprising:
reacting a compound of Formula 33, 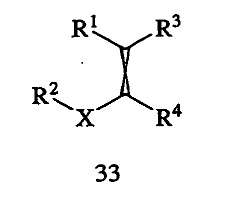 with hydrogen in the presence of a chiral catalyst to yield the compound of Formula 32; and
optionally converting the compound of Formula 32 into a pharmaceutically acceptable complex, salt, solvate or hydrate;
wherein the chiral catalyst comprises a chiral ligand bound to a transition metal through phosphorus atoms, the chiral ligand having a structure represented by Formula 4, 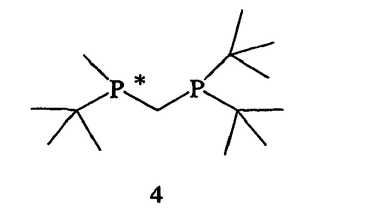 and wherein R¹, R², R³, R⁴, and X in Formula 3 are as defined in Formula 2.

8. The method of any one of claims 1 to 3 and 7, wherein Y is a Group 1 metal ion, a Group 2 metal ion, a primary ammonium ion, or a secondary ammonium ion.

9. The method of any one of claims 1 to 8, wherein the transition metal is rhodium.

10. The method of any one of claims 1 to 9, wherein the chiral ligand comprises an enantiomer having a structure represented by Formula 5, 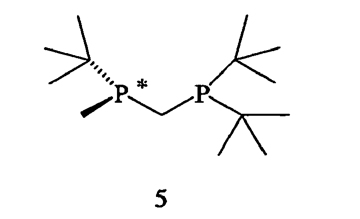 and an ee of about 95 % or greater.

11. A method of making a desired enantiomer of a compound of Formula 4, 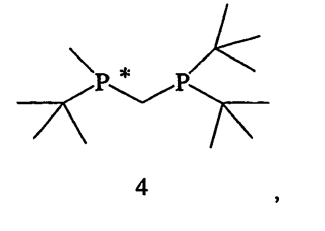 the method comprising:
reacting a compound of Formula 9, 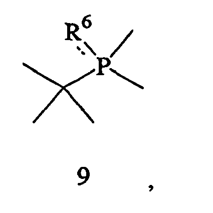 with a compound of Formula 10, 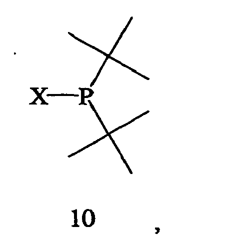 to yield a compound of Formula 11, 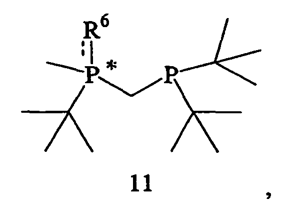
wherein the compound of Formula 9 is treated with a base prior to reaction with the compound of Formula 10, X is a leaving group, and R⁶ is BH₃, sulfur, or oxygen; and
reacting the compound of Formula 11 with a borane, sulfur, or oxygen to yield a compound of Formula 12, 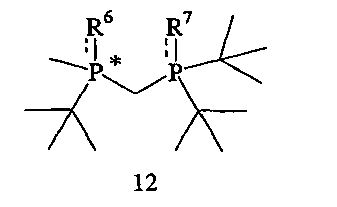 wherein R⁷ is the same as or different than R⁶ and is BH₃, sulfur, or oxygen; and removing R⁶ and R⁷ from the compound of Formula 12 to yield the compound of Formula 4, wherein the compound of Formula 12 is resolved into separate enantiomers before or after removal of R⁶ and R⁷.

12. The method of claim 11, wherein the desired enantiomer has R-absolute stereochemical configuration.

13. The method of claim 11, wherein removing R⁶ and R⁷ comprises reacting a compound of Formula 13, 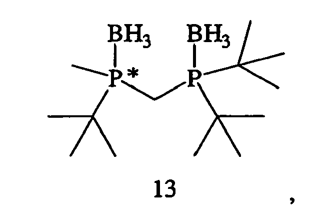 with an amine or an acid to yield the compound of Formula 4; or
treating the compound of Formula 12 with a reducing agent when R⁶ and R⁷ are each sulfur or oxygen; or
reacting a compound of Formula 14, 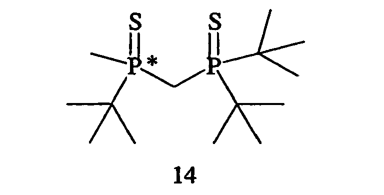 with R⁸OTf to yield a compound of Formula 15, 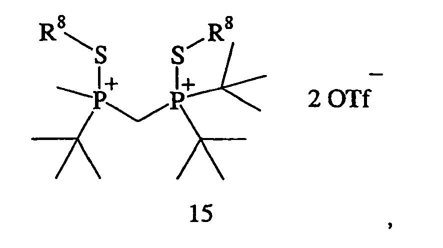 in which R⁸ is a C₁₋₄ alkyl;
reacting the compound of Formula 15 with a borohydride to yield the compound of Formula 13, 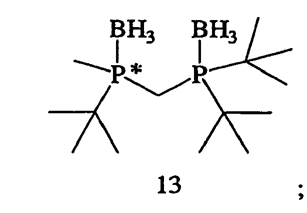 and either
reacting the compound of Formula 13 with an amine or an acid to yield the compound of Formula 4; or '
reacting the compound of Formula 13 with HCl to yield a compound of Formula 15, 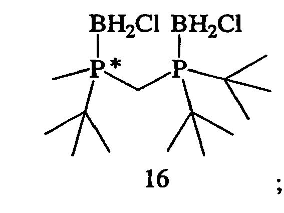 and
reacting the compound of Formula 16 with an amine or an acid to yield the compound of Formula 4.

14. A compound of Formula 19, 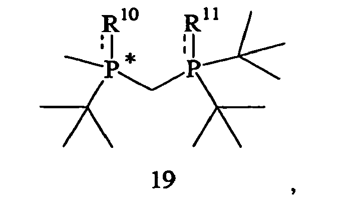 in which R¹⁰ and R¹¹ are independently BH₃, BH₂Cl, sulfur, oxygen, C₁₋₄ alkylthio, or absent, and subject to the proviso that R¹⁰ and R¹¹ are not both BH₃.

15. The compound of claim 14, selected from:
2-{ [(di-*t*-butyl-phosphanyl)-methyl]-methyl-phosphanyl }-2-methyl-propane;
(*R*)-2-{ [(di-*t*-butyl-phosphanyl)-methyl]-methyl-phosphanyl}-2-methyl-propane;
(*S*)-2- {[(di-*t*-butyl-phosphanyl)-methyl]-methyl-phosphanyl}-2-methyl-propane;
2-[(di-*t*-butyl-phosphinothioylmethyl)-methyl-phosphinothioyl]-2-methyl-propane;
(R)-2-[(di-*t*-butyl-phosphinothioylmethyl)-methyl-phosphinothioyl]-2-methyl-propane;
(*S*)-2-[(di-*t*-butyl-phosphinothioylmethyl)-methyl-phosphinothioyl]-2-methyl-propane;
2-[(di-*t*-butyl-phosphinoylmethyl)-methyl-phosphinoyl]-2-methyl-propane;
(*R*)-2-[(di-*t*-butyl-phosphinoylmethyl)-methyl-phosphinoyl]-2-methyl-propane;
(*S*)-2-[(di-*t*-butyl-phosphinoylmethyl)-methyl-phosphinoyl]-2-methyl-propane;
(di-t butyl-methylthio-phosphoniumyl-methyl)-t-butyl-methyl-methylthio-phosphonium;
(*R*)-(di-*t*-butyl-methylthio-phosphoniumyl-methyl)-*t*-butyl-methyl-methylthio-phosphonium; or
(*S*)-(di-*t*-butyl-methylthio-phosphoniumyl-methyl)-*t*-butyl-methyl-methylthio-phosphonium.

## Patentansprüche

1. Verfahren zum Herstellen eines gewünschten Enantiomers einer Verbindung der Formel 2 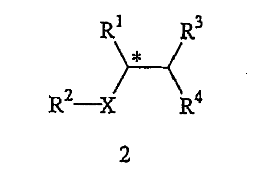 oder eines pharmazeutisch annehmbaren Komplexes, Salzes, Solvates oder Hydrates davon, worin
R¹ C₁₋₆-Alkyl, C₁₋₇-Alkanoylamino, C₁₋₆-Alkoxycarbonyl, C₁₋₆-Alkoxycarbonylamino, Amino, Amino-C₁₋₆-alkyl, C₁₋₆-Alkylamino, Cyano, Cyano-C₁₋₆-alkyl, Carboxy oder -CO₂-Y ist;
R² C₁₋₇-Alkanoyl, C₁₋₆-Alkoxycarbonyl, Carboxy oder -CO₂-Y ist;
R³ und R⁴ unabhängig Wasserstoffatom, C₁₋₆-Alkyl, C₃₋₇-Cycloalkyl, Aryl, Aryl-C₁₋₆-alkyl sind oder R³ und R⁴ zusammen C₂₋₆-Alkandiyl sind;
X -NH-, -O-, -CH₂- oder eine Bindung ist; und
Y ein Kation ist;
wobei das Verfahren umfasst:
Umsetzen einer Verbindung der Formel 3 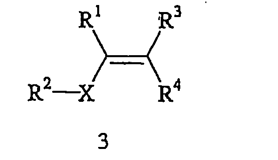 mit Wasserstoff in Gegenwart eines chiralen Katalysators unter Erhalt der Verbindung der Formel 2; und
gegebenenfalls Umwandeln der Verbindung der Formel 2 in ein pharmazeutisch annehmbares Salz, einen pharmazeutisch annehmbaren Komplex, ein pharmazeutisch annehmbares Solvat oder Hydrat;
wobei der chirale Katalysator einen chiralen Liganden, gebunden an ein Übergangsmetall durch Phosphoratome, umfasst, wobei der chirale Ligand eine Struktur hat, dargestellt durch Formel 4 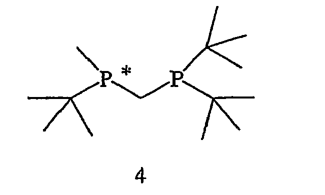 und worin R¹, R², R³, R⁴ und X in Formel 3 wie in Formel 2 definiert sind.

2. Verfahren zum Herstellen einer Verbindung der Formel 1 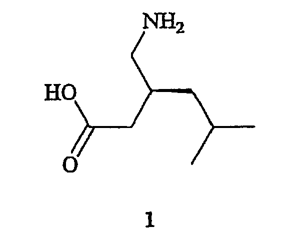 oder eines pharmazeutisch annehmbaren Komplexes, Salzes, Solvates oder Hydrates davon, wobei das Verfahren umfasst:
Umsetzen einer Verbindung der Formel-6 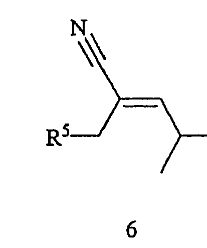 eines entsprechenden Z-Isomers der Verbindung der Formel 6 oder eines Gemisches davon mit Wasserstoff in Gegenwart eines chiralen Katalysators unter Erhalt einer Verbindung der Formel 7 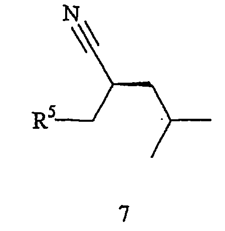
worin R⁵ eine Carboxygruppe oder -CO₂-Y ist, Y ein Kation ist und der chirale Katalysator einen chiralen Liganden, gebunden an ein Übergangsmetall durch Phosphoratome, umfasst, wobei der chirale Ligand eine Struktur hat, dargestellt durch Formel 4 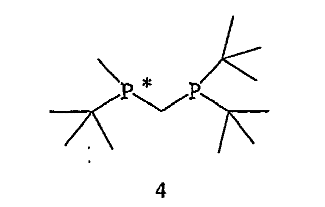 Reduzieren einer Cyanogruppierung der Verbindung der Formel 7 unter Erhalt einer Verbindung der Formel 8 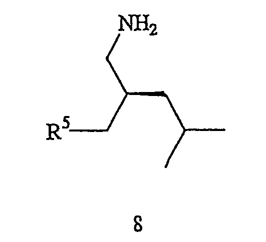 gegebenenfalls Behandeln der Verbindung der Formel 8 mit einer Säure unter Erhalt der Verbindung der Formel 1; und
gegebenenfalls Umwandeln der Verbindung der Formel 8 oder der Formel 1 in einen pharmazeutisch annehmbaren Komplex, ein pharmazeutisch annehmbares Salz, ein pharmazeutisch annehmbares Solvat oder Hydrat.

3. Verfahren nach Anspruch 2, wobei die Verbindung der Formel 6 ein Basenadditionssalz von 3-Cyano-5-methylhex-3-ensäure ist.

4. Verfahren nach Anspruch 3, wobei die Verbindung der Formel 6 3-Cyano-5-methylhex-3-enoat-t-butyl-ammoniumsalz ist.

5. Verfahren zum Herstellen eines Katalysators oder Präkatalysators, bestehend aus einem chiralen Liganden, gebunden an ein Übergangsmetall durch Phosphoratome, wobei der chirale Ligand eine Struktur hat, dargestellt durch Formel 4 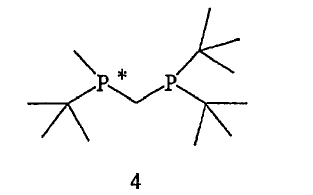 wobei das Verfahren umfasst:
Entfernen des Substituenten R⁹ von einer Verbindung der Formel 17 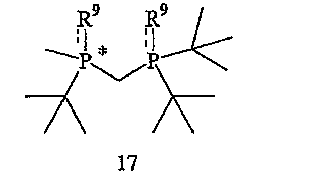 unter Erhalt einer Verbindung der Formel 4, wobei R⁹ BH₃, Schwefel oder Sauerstoff ist; und
Binden der Verbindung der Formel 4 an ein Übergangsmetall.

6. Katalysator oder Präkatalysator, umfassend einen chiralen Liganden, gebunden an ein Übergangsmetall durch Phosphoratome, wobei der chirale Ligand eine Struktur hat, dargestellt durch Formel 4

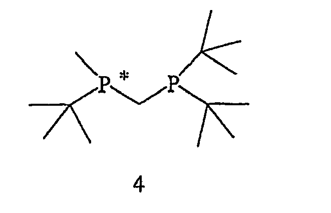

7. Verfahren zum Herstellen eines gewünschten Enantiomers einer Verbindung der Formel 32 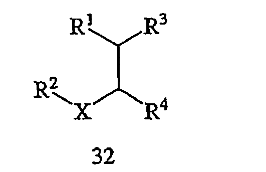 oder eines pharmazeutisch annehmbaren Komplexes, Salzes, Solvates oder Hydrates davon, worin
R¹ C₁₋₆-Alkyl, C₁₋₇-Alkanoylamino, C₁₋₆-Alkoxycarbonyl, C₁₋₆-Alkoxycarbonylamino, Amino, Amino-C₁₋₆-alkyl, C₁₋₆-Alkylamino, Cyano, Cyano-C₁₋₆-alkyl, Carboxy oder -CO₂-Y ist;
R² C₁₋₇-Alkanoyl, C₁₋₆-Alkoxycarbonyl, Carboxy oder -CO₂-Y ist;
R³ und R⁴ unabhängig Wasserstoffatom, C₁₋₆-Alkyl, C₃₋₇-Cycloalkyl, Aryl, Aryl-C₁₋₆-alkyl sind oder R³ und R⁴ zusammen C₂₋₆-Alkandiyl sind;
X -NH-, -O-, -CH₂- oder eine Bindung ist; und
Y ein Kation ist;
wobei das Verfahren umfasst:
Umsetzen einer Verbindung der Formel 33 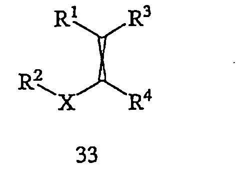 mit Wasserstoff in Gegenwart eines chiralen Katalysators unter Erhalt der Verbindung der Formel 32; und
gegebenenfalls Umwandeln der Verbindung der Formel 32 in einen pharmazeutisch annehmbaren Komplex, ein pharmazeutisch annehmbares Salz, Solvat oder Hydrat;
wobei der chirale Katalysator einen chiralen Liganden, gebunden an ein Übergangsmetall durch Phosphoratome, umfasst, wobei der chirale Ligand eine Struktur hat, dargestellt durch Formel 4 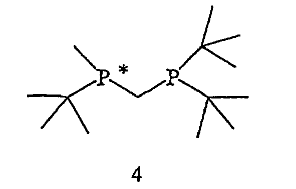 und worin R¹, R², R³, R⁴ und X in Formel 3 wie in Formel 2 definiert sind.

8. Verfahren nach einem der Ansprüche 1 bis 3 und 7, wobei Y ein Gruppe 1-Metallion, ein Gruppe 2-Metallion, ein primäres Ammoniumion oder ein sekundäres Ammoniumion ist.

9. Verfahren nach einem der Ansprüche 1 bis 8, wobei das Übergangsmetall Rhodium ist.

10. Verfahren nach einem der Ansprüche 1 bis 9, wobei der chirale Ligand ein Enantiomer umfasst, das eine Struktur hat, dargestellt durch Formel 5 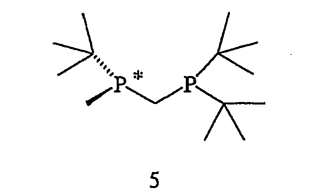 und einen ee von etwa 95% oder größer.

11. Verfahren zum Herstellen eines gewünschten Enantiomers einer Verbindung der Formel 4 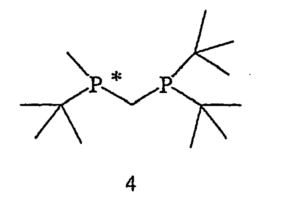 wobei das Verfahren umfasst:
Umsetzen einer Verbindung der Formel 9 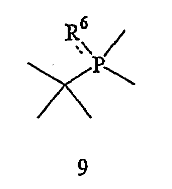 mit einer Verbindung der Formel 10, 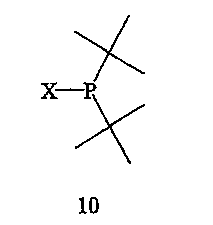 unter Erhalt einer Verbindung der Formel 11 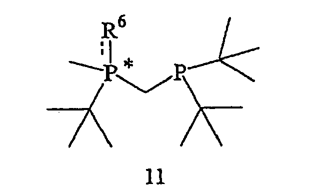
wobei die Verbindung der Formel 9 mit einer Base vor der Reaktion mit der Verbindung der Formel 10 behandelt wird, X eine Abgangsgruppe ist und R⁶ BH₃, Schwefel oder Sauerstoff ist; und
Umsetzen der Verbindung der Formel 11 mit einem Boran, Schwefel oder Sauerstoff unter Erhalt einer Verbindung der Formel 12 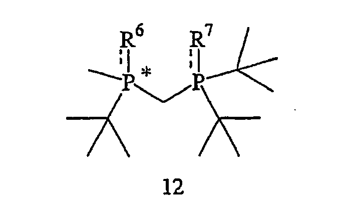 worin R⁷das gleiche ist wie oder verschieden von R⁶ und BH₃ Schwefel oder Sauerstoff ist; und
Entfernen von R⁶ und R⁷ von der Verbindung der Formel 12 unter Erhalt der Verbindung der Formel 4, wobei die Verbindung der Formel 12 vor oder nach dem Entfernen von R⁶ und R⁷ in getrennte Enantiomere getrennt wird.

12. Verfahren nach Anspruch 11, wobei das gewünschte Enantiomer absolute stereochemische R-Konfiguration hat.

13. Verfahren nach Anspruch 11, wobei das Entfernen von R⁶ und R⁷ das Umsetzen einer Verbindung der Formel 13 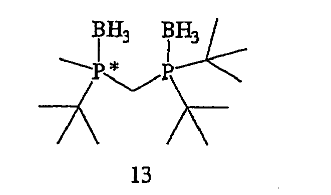 mit einem Amin oder einer Säure unter Erhalt der Verbindung der Formel 4; oder
das Behandeln der Verbindung der Formel 12 mit einem Reduktionsmittel, wenn R⁶ und R⁷ jeweils Schwefel oder Sauerstoff sind; oder
das Umsetzen einer Verbindung der Formel 14 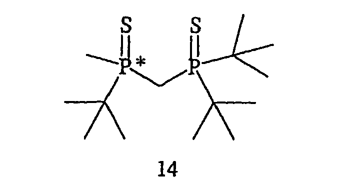 mit R⁸OTf unter Erhalt einer Verbindung der Formel 15 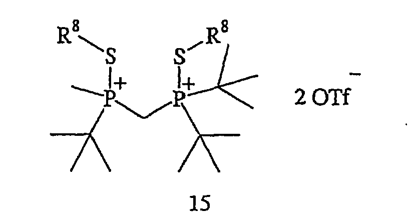 worin R⁸ ein C₁₋₄-Alkyl ist;
das Umsetzen der Verbindung der Formel 15 mit einem Borhydrid unter Erhalt der Verbindung der Formel 13 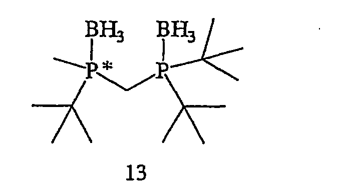 und entweder
das Umsetzen der Verbindung der Formel 13 mit einem Amin oder einer Säure unter Erhalt der Verbindung der Formel 4; oder
das Umsetzen der Verbindung der Formel 13 mit HCl unter Erhalt einer Verbindung der Formel 15 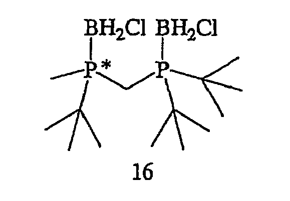 und
das Umsetzen der Verbindung der Formel 16 mit einem Amin oder einer Säure unter Erhalt der Verbindung der Formel 4, umfasst.

14. Verbindung der Formel 19 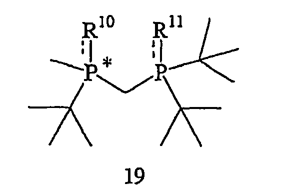 worin R¹⁰ und R¹¹ unabhängig BH₃, BH₂Cl, Schwefel, Sauerstoff, C₁₋₄-Alkylthio oder nicht vorhanden und Gegenstand der Maßgabe sind, dass R¹⁰ und R¹¹ nicht beide BH₃ sind.

15. Verbindung nach Anspruch 14, ausgewählt aus:
2-{[(Di-t-butyl-phosphanyl)-methyl]-methyl-phosphanyl}-2-methyl-propan;
(R)-2-{[(Di-t-butyl-phosphanyl)-methyl]-methyl-phosphan-yl}-2-methyl-propan;
(S)-2-{[(Di-t-butyl-phosphanyl)-methyl]-methyl-phosphan-yl]}-2-methyl-propan;
2-[(Di-t-butyl-phosphinothioylmethyl)-methyl-phosphino-thioyl]-2-methyl-propan;
(R)-2-[(Di-t-butyl-phosphinothioylmethyl)-methyl-phos-phinothioyl]-2-methyl-propan;
(S)-2-[(Di-t-butyl-phosphinothioylmethyl)-methyl-phos-phinothioyl]-2-methyl-propan;
2-[(Di-t-butyl-phosphinoylmethyl)-methyl-phosphinoyl]-2-methyl-propan;
(R)-2-[(Di-t-butyl-phosphinoylmethyl)-methyl-phosphino-yl]-2-methyl-propan;
(S)-2-[(Di-t-butyl-phosphinoylmethyl)-methyl-phosphinoyl]-2-methyl-propan;
(Di-t-butyl-methylthio-phosphoniumyl-methyl)-t-butylmethyl-methylthio-phosphonium;
(R)-(Di-t-butyl-methylthio-phosphoniumyl-methyl)-t-butylmethyl-methylthio-phosphonium; oder
(S)-(Di-t-butyl-methylthio-phosphoniumyl-methyl)-t-butylmethyl-methylthio-phosphonium.

## Revendications

1. Procédé pour la préparation d'un énantiomère désiré d'un composé de formule 2 : 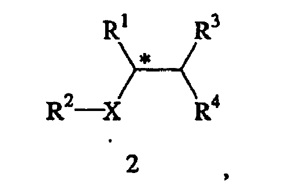 ou d'un de ses complexes, sels, produits de solvatation ou hydrates pharmaceutiquement acceptables, formule dans laquelle :
R¹ représente un groupe alkyle en C₁ à C₆, alcanoylamino en C₁ à C₇, (alkoxy en C₁ à C₆) carbonyle, (alkoxy en C₁ à C₆)carbonylamino, amino, aminoalkyle en C₁ à C₆, alkylamino en C₁ à C₆, cyano, cyano (alkyle en C₁ à C₆) , carboxy ou -CO₂-Y ;
R² représente un groupe alcanoyle en C₁ à C₇, (alkoxy en C₁ à C₆) carbonyle, carboxy ou -CO₂-Y ;
R³ et R⁴ représentent indépendamment un atome d'hydrogène, un groupe alkyle en C₁ à C₆, cycloalkyle en C₃ à C₇, aryle ou aryl (alkyle en C₁ à C₆), ou bien R³ et R⁴, conjointement, représentent un groupe alcanediyle en C₂ à C₆ ;
X représente un groupe -NH-, -O-, -CH₂- ou une liaison ; et
Y représente un cation ;
le procédé comprenant :
la réaction d'un composé de formule 3 : 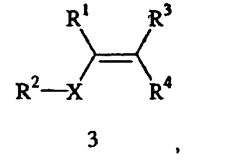 avec de l'hydrogène en présence d'un catalyseur chiral pour donner le composé de formule 2 ; et
facultativement la conversion du composé de formule 2 en un sel, complexe, produit de solvatation ou hydrate pharmaceutiquement acceptable ;
dans lequel le catalyseur chiral comprend un ligand chiral lié à un métal de transition par des atomes de phosphore, le ligand chiral ayant une structure représentée par la formule 4 : 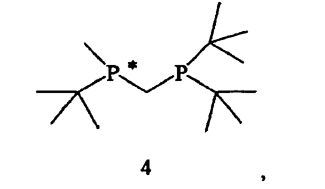 les groupes R¹, R², R³, R⁴ et Y dans la formule 3 répondant aux définitions mentionnées pour la formule 2.

2. Procédé pour la préparation d'un composé de formule 1 : 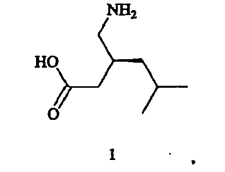 ou d'un de ses complexes, sels, produits de solvatation ou hydrates pharmaceutiquement acceptables, le procédé comprenant :
la réaction d'un composé de formule 6 : 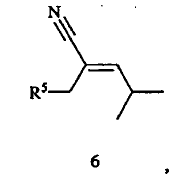 d'un isomère Z correspondant du composé de formule 6, ou d'un de leurs mélanges, avec de l'hydrogène en présence d'un catalyseur chiral pour donner un composé de formule 7 : 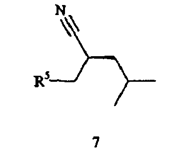 dans laquelle R⁵ représente un groupe carboxy ou -CO₂-Y, Y représente un cation, et le catalyseur chiral comprend un ligand chiral lié à un métal de transition par des atomes de phosphore, le ligand chiral ayant une structure représentée par la formule 4 : 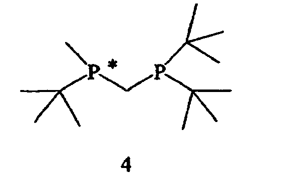
la réduction d'un groupement cyano du composé de formule 7 pour donner un composé de formule 8 : 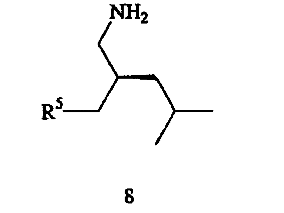
facultativement le traitement du composé de formule 8 avec un acide pour donner le composé de formule 1 ; et
facultativement la conversion du composé de formule 8 ou de formule 1 en un complexe, sel, produit de solvatation ou hydrate pharmaceutiquement acceptable.

3. Procédé suivant la revendication 2, dans laquelle le composé de formule 6 est un sel d'addition de base de l'acide 3-cyano-5-méthyl-hex-3-énoïque.

4. Procédé suivant la revendication 3, dans lequel le composé de formule 6 est le sel 3-cyano-5-méthyl-hex-3-énoate de tertiobutylammonium.

5. Procédé pour la préparation d'un catalyseur ou précatalyseur constitué d'un ligand chiral lié à un métal de transition par des atomes de phosphore, le ligand chiral ayant une structure représentée par la formule 4 : 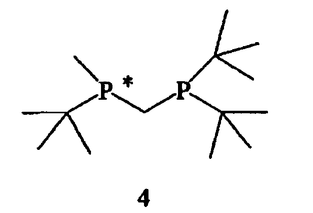 le procédé comprenant :
l'élimination du substituant R⁹ d'un composé de formule 17 : 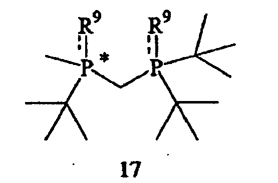 pour donner un composé de formule 4, le substituant R⁹ consistant en BH₃, soufre ou oxygène ; et la liaison du composé de formule 4 à un métal de transition.

6. Catalyseur ou précatalyseur comprenant un ligand chiral lié à un métal de transition par des atomes de phosphore, le ligand chiral ayant une structure représentée par la formule 4 :

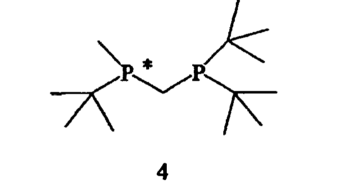

7. Procédé pour la préparation d'un énantiomère désiré d'un composé de formule 32 : 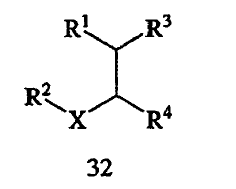 ou d'un de ses complexes, sels, produits de solvatation ou hydrates pharmaceutiquement acceptables, formule dans laquelle :
R¹ représente un groupe alkyle en C₁ à C₆, alcanoylamino en C₁ à C₇, (alkoxy en C₁ à C₆)carbonyle, (alkoxy en C₁ à C₆)carbonylamino, amino, aminoalkyle en C₁ à C₆, alkylamino en C₁ à C₆, cyano, cyano (alkyle en C₁ à C₆), carboxy ou -CO₂-Y ;
R² représente un groupe alcanoyle en C₁ à C₇, (alkoxy en C₁ à C₆)carbonyle, carboxy ou -CO₂-Y;
R³ et R⁴ représentent indépendamment un atome d'hydrogène, un groupe alkyle en C₁ à C₆, cycloalkyle en C₃ à C₇, aryle ou aryl (alkyle en C₁ à C₆), ou bien R³ et R⁴, conjointement, représentent un groupe alcanediyle en C₂ à C₆;
X représente un groupe -NH-, -O-, -CH₂- ou une liaison ; et
Y représente un cation ;
le procédé comprenant :
la réaction d'un composé de formule 33 : 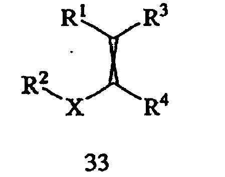 avec de l'hydrogène en présence d'un catalyseur chiral pour donner le composé de formule 32 ; et
facultativement la conversion du composé de formule 32 en un complexe, sel, produit de solvatation ou hydrate pharmaceutiquement acceptable ;
dans lequel le catalyseur chiral comprend un ligand chiral lié à un métal de transition par des atomes de phosphore, le ligand chiral ayant une structure représentée par la formule 4 : 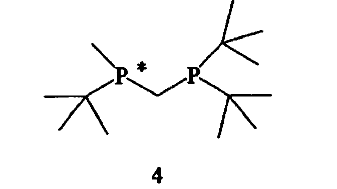 les groupes R¹, R², R³, R⁴ et X dans la formule 3 étant tels que définis dans la formule 2.

8. Procédé suivant l'une quelconque des revendications 1 à 3 et 7, dans lequel Y représente un ion de métal du Groupe 1, un ion de métal du Groupe 2, un ion ammonium primaire ou un ion ammonium secondaire.

9. Procédé suivant l'une quelconque des revendications 1 à 8, dans lequel le métal de transition est le rhodium.

10. Procédé suivant l'une quelconque des revendications 1 à 9, dans lequel le ligand chiral comprend un énantiomère ayant une structure représentée par la formule 5 : 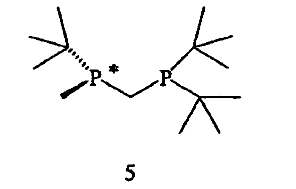 et un ee approximativement égal ou supérieur à 95 %.

11. Procédé pour la préparation d'un énantiomère désiré d'un composé de formule 4 : 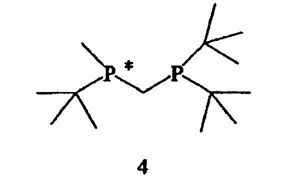 le procédé comprenant :
la réaction d'un composé de formule 9 : 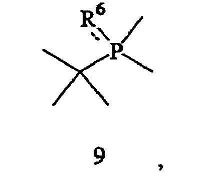 avec un composé de formule 10 : 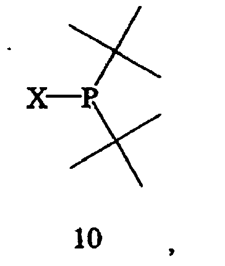 pour donner un composé de formule 11 : 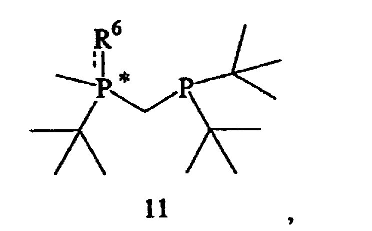 dans lequel le composé de formule 9 est traité avec une base avant réaction avec le composé de formule 10, X représente un groupe partant, et R⁶ représente BH₃, le soufre ou l'oxygène ; et
la réaction du composé de formule 11 avec un borane, du soufre ou de l'oxygène pour donner un composé de formule 12 : 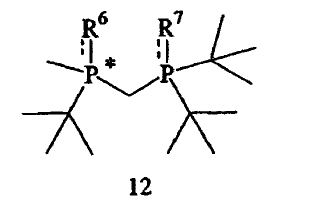 dans laquelle R⁷ est identique à, ou différent de, R⁶ et représente BH₃, le soufre ou l'oxygène ; et
l'élimination de R⁶ et R⁷ du composé de formule 12 pour donner le composé de formule 4, le composé de formule 12 étant soumis à une résolution en des énantiomères séparés avant ou après l'élimination de R⁶ et R⁷.

12. Procédé suivant la revendication 11, dans lequel l'énantiomère désiré a la configuration stéréochimique absolue R.

13. Procédé suivant la revendication 11, dans lequel l'élimination de R⁶ et R⁷ comprend la réaction d'un composé de formule 13 : 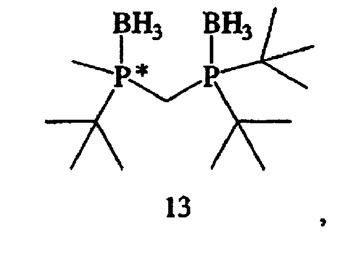 avec une amine ou un acide pour donner le composé de formule 4 ; ou
le traitement du composé de formule 12 avec un agent réducteur lorsque R⁶ et R⁷ représentent chacun le soufre ou l'oxygène ; ou
la réaction d'un composé de formule 14 : 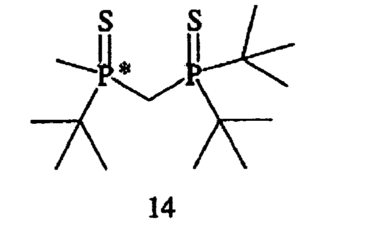 avec R⁸OTf pour donner le composé de formule 15 : 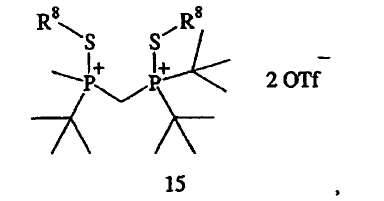 dans laquelle R⁸ représente un groupe alkyle en C₁ à C₄ ;
la réaction du composé de formule 15 avec un borohydrure pour donner le composé de formule 13 : 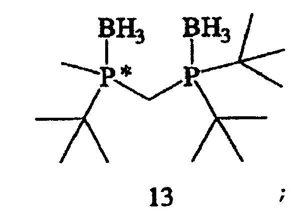 et soit
la réaction du composé de formule 13 avec une amine ou un acide pour donner le composé de formule 4 ; soit
la réaction du composé de formule 13 avec HCl pour donner un composé de formule 15 : 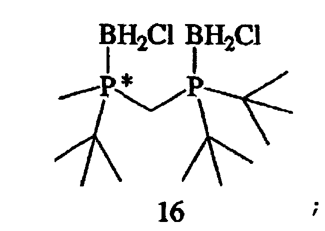 et
la réaction du composé de formule 16 avec une amine ou un acide, pour donner le composé de formule 4.

14. Composé de formule 19 : 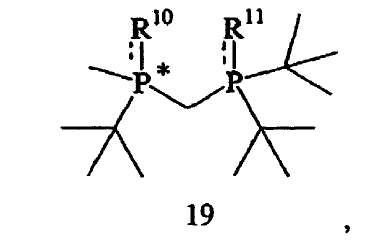 dans laquelle R¹⁰ et R¹¹ représentent indépendamment BH₃, BH₂Cl, le soufre, l'oxygène, un groupe alkylthio en C₁ à C₄, ou bien sont absents, sous réserve que R¹⁰ et R¹¹ ne représentent ni l'un ni l'autre BH₃.

15. Composé suivant la revendication 14, choisi entre :
le 2-{[(ditertiobutyl-phosphanyl)-méthyl]-méthyl-phosphanyl}-2-méthyl-propane ;
le (*R*)-2-{[(ditertiobutyl-phosphanyl)-méthyl]-méthyl-phosphanyl}-2-méthyl-propane ;
le (*S*)-2-{[(ditertiobutyl-phosphanyl)-méthyl]-méthyl-phosphanyl}-2-méthyl-propane ;
le 2-[(ditertiobutyl-phosphinothioylméthyl)-méthyl-phosphinothioyl]-2-méthyl-propane ;
le (*R*)-2-[(ditertiobutyl-phosphinothioylméthyl)-méthyl-phosphinothioyl]-2-méthyl-propane ;
le (*S*) -2- (ditertiobutyl-phosphinothioylméthyl)-méthyl-phosphinothioyl]-2-méthyl-propane ;
le 2-[(ditertiobutyl-phosphinoylméthyl)-méthyl-phosphinoyl]-2-méthyl-propane ;
le (*R*)-2-[(ditertiobutyl-phosphinoylméthyl)-méthyl-phosphinoyl]-2-méthyl-propane ;
le (*S*)-2-[(ditertiobutyl-phosphinoylméthyl)-méthyl-phosphinoyl]-2-méthyl-propane ;
le (ditertiobutyl-méthylthio-phosphoniumyl-méthyl)-tertiobutyl-méthyl-méthylthio-phosphonium ;
le (R)-(ditertiobutyl-méthylthio-phosphoniumyl-méthyl)-tertiobutyl-méthyl-méthylthio-phosphonium ; et
le (*S*)-(ditertiobutyl-méthylthio-phosphoniumyl-méthyl)-tertiobutyl-méthyl-méthylthio-phosphonium.
